# EUROPÄISCHE PATENTSCHRIFT

(11) **EP 1 311 476 B1**
(45) Veröffentlichungstag und Bekanntmachung des Hinweises auf die Patenterteilung: **16.03.2005**
(21) Anmeldenummer: 01960693.8
(22) Anmeldetag: 21.08.2001
(51) Int. Cl.: C07C 311/47, C07C 317/42, C07C 333/04, C07D 211/46, C07D 213/30, C07D 257/04, C07D 295/08, C07D 317/40, A61K 31/175, A61K 31/18, A61P 9/00, A61P 35/00

(54) **AZA-AMINOSÄUREDERIVATE (FAKTOR X a?-INHIBITOREN 15)**
AZA-AMINO ACID DERIVATIVES (FACTOR X A?-INHIBITORS 15)
DERIVES D'ACIDES AMINES AZA (INHIBITEURS 15 DU FACTEUR XA)

(30) Priorität: 21.08.2000 DE 10040783
(43) Veröffentlichungstag der Anmeldung: 21.05.2003
(73) Patentinhaber: MERCK PATENT GmbH, 64293 Darmstadt (DE)
(72) Erfinder: MEDERSKI, Werner, 64390 Erzhausen (DE); JURASZYK, Horst, 64342 Seeheim-Jugenheim (DE); DORSCH, Dieter, 64372 Ober-Ramstadt (DE); TSAKLAKIDIS, Christos, 69469 Weinheim (DE); GLEITZ, Johannes, 64293 Darmstadt (DE); BARNES, Christopher, 65812 Bad Soden (DE)
(86) Internationale Anmeldenummer: PCT/EP2001/009667
(87) Internationale Veröffentlichungsnummer: WO 2002/016315

(56) Entgegenhaltungen:
- EP-A- 1 020 434
- J.M. FEVIG, ET AL.: "Preparation of meta-amidino-N,N-disubstituted anilines as potent inhibitors of coagulation factor Xa" BIOORGANIC AND MEDICINAL CHEMISTRY LETTERS, Bd. 8, Nr. 22, 17. November 1998 (1998-11-17), Seiten 3143-3148, XP004143716 Elsevier Science Publishers, Amsterdam, NL ISSN: 0960-894X

## Beschreibung

Die Erfindung betrifft Semicarbazide der allgemeinen Formel **I**, 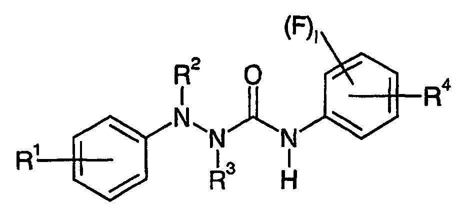 wobei bedeutet:
- R¹: -(CH₂)ₙ-NH₂, -CON=C(NH₂)₂, -NHC(=NH)-NH₂ oder -C(=NH)-NH₂, das auch einfach mit -OH, -OCOOA, -OCOO(CH₂)ₙN(A)₂, -OCOO(CH₂)ₘ-Het, -CO-C(A)₂-R⁵, -COOA, -COSA, -COOAr, -COOAr' oder durch 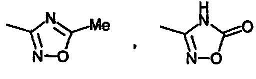 substituiert sein kann,
- R²: H, COOA,
- R³: unverzweigtes, verzweigtes oder cyclisches Alkyl mit 1-20 C-Atomen, worin eine oder zwei CH₂-Gruppen durch O- oder S-Atome ersetzt sein können, Ar, Ar', X oder Hal,
- R⁴: mit S(O)ₖA, S(O)ₖNHA, CF₃, COOA, CH₂NHA, CN oder OA monosubstituiertes Phenyl,
- R⁵: -CHal₃, -O(C=O)A oder 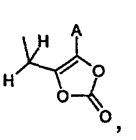
- Ar: unsubstituiertes oder ein-, zwei- oder dreifach durch A, OH, OA, NH₂, NHA, NA₂, NO₂, CF₃, CN, Hal, NHCOA, COOA, CONH₂, CONHA, CONA₂, S(O)ₙA, S(O)ₙNH₂, S(O)ₙNHA, S(O)ₙNA₂ substituiertes Phenyl oder Naphthyl,
- Ar': -(CH₂)ₙ-Ar,
- Het: einen ein- oder zweikernigen gesättigten, ungesättigten oder aromatischen Heterocyclus mit 1 bis 4 N-, O- und/oder S-Atomen, über N oder C gebunden, der unsubstituiert oder durch A substituiert sein kann,
- A: H, unverzweigtes, verzweigtes oder cyclisches Alkyl mit 1-20 C-Atomen,
- X: -(CH₂)ₙ-Y,
- Y: COOA, 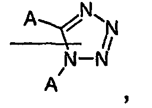
- Hal: F, Cl, Br oder I,
- n: 1, 2, 3, 4, 5 oder 6,
- m: 0 oder 1,
- k: 0, 1 oder 2,
- l: 0, 1, 2, 3 oder 4,
sowie ihre pharmazeutisch verträglichen Salze und Solvate.

Gegenstand der Erfindung sind auch die optisch aktiven Formen, die Racemate, die Diastereomeren sowie die Hydrate und Solvate, z.B. Alkoholate dieser Verbindungen.

Zur Bekämpfung von durch Verletzungen verursachten Blutungen besitzt der menschliche Organismus einen Mechanismus, durch den mit Hilfe von Blutgerinnseln ein schneller Wundverschluss erreicht wird. Blutgerinnsel bilden sich durch eine Serie von Zymogenaktivierungen. Im Verlauf dieser enzymatischen Kaskade katalysiert jeweils die aktivierte Form eines Faktors die Aktivierung des nächsten. Da dieser Prozeß katalytischer Natur ist, genügen kleinste Mengen des auslösenden Faktors, um die Kaskade in Gang zu setzen. Durch die Vielzahl der Schritte wird eine große Verstärkung erreicht, die eine schnelle Antwort auf die Verletzung gewährleistet. Die plasmatische Gerinnung nach einer Gewebsläsion kann auf exogenem Weg durch die Freisetzung von Gewebsthrombokinase erfolgen. Die entsprechende Reaktionsfolge wird als extravaskuläres System (Extrinsik-System) bezeichnet und läuft innerhalb von Sekunden ab. Die Gerinnung kann auch auf endogenem Weg durch Thrombozytenzerfall ausgelöst werden. Diese Reaktionsfolge, die als intravaskuläres System bezeichnet wird, läuft innerhalb von Minuten ab. Beide Systeme münden in eine abschließende gemeinsame Folge von Schritten, die zur Bildung eines Fibringerinnsels führen. Das intravaskuläre und das extravaskuläre System beeinflussen sich in vivo gegenseitig. Beide sind für den vollständigen Ablauf der Blutgerinnung notwendig.

So wichtig eine schnelle Blutgerinnung für den Verschluß von Verletzungen ist, ist es doch bei bestimmten Erkrankungen erforderlich, die Blutgerinnung zu hemmen um z.B. die Bildung von Thromben in Gefäßen zu vermeiden.

Dabei sollte möglichst gezielt und selektiv in die Blutgerinnungskaskade eingegriffen werden um die Inhibierung möglichst genau steuern zu können und unerwünschte Nebenwirkungen vermeiden zu können.

Faktor Xₐ ist eine Serinprotease der Blutgerinnungskaskade, welche durch Aktivierung des Faktors X gebildet wird. Diese Aktivierung erfolgt beim intravaskulären Weg durch den Faktor IXₐ, wobei diese Reaktion durch den antihämophilen Faktor (VIIIₐ) stimuliert wird. Durch den Faktor Xₐ wird anschließend Prothrombin in Thrombin umgewandelt. Das proteolytische Enzym Thrombin spaltet Fibrinogen in Fibrinmonomere, die sich spontan zu geordneten faserförmigen Strukturen zusammenlagern, die man als Fibrin bezeichnet. Das Gerinnsel, das durch die spontane Aggregation von Fibrinmonomeren entsteht, wird durch kovalente Quervernetzungen zwischen den Seitenketten verschiedener Moleküle in den Fibrinfasern stabilisiert. Dazu bilden sich zwischen spezifischen Glutamin- und Lysinseitenketten in einer Transamidierungsreaktion Peptidbindungen. Diese Quervernetzung wird durch ein Enzym katalysiert, das man als Faktor XIIIₐ bezeichnet.

Beim extravaskulären System erfolgt die Aktivierung des Faktors X durch den Gewebsfaktor sowie den Faktor VII.

Eine Inhibierung des Faktor Xₐ erlaubt einen gezielten Eingriff in die Blutgerinnung, da hierbei keine anderen Prozesse beeinflußt werden. Dies ist vorteilhafter als beispielsweise eine Inhibierung von Thrombin, da Thrombin einerseits die Umwandlung von Fibrinogen zu Fibrin katalysiert, wie auch die Umwandlungen von Faktor VIII in VIIIₐ, Faktor V in Vₐ sowie Faktor XI in XIₐ, und andererseits beispielsweise auch Thrombozyten aktiviert. Es sind daher vielfältige Forschungsaktivitäten zur Entwicklung von Inhibitoren des Faktors Xₐ unternommen worden, die zur Entwicklung diverser Substanzklassen geführt haben.

In der WO 99/11657 werden 1-Amino-7-isochinolinderivate beschrieben, die als Inhibitoren von Serinproteasen wirken. In der WO 99/11658 werden m-Benzamidinderivate beschrieben, welche als Serinprotease-Inhibitoren wirken. Weiterhin werden in der WO 99/10316 3-Amidinoanilinderivate beschrieben, die als Inhibitoren des aktivierten Blutgerinnungsfaktors Xₐ wirken.

Aufgabe der Erfindung ist es, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie Faktor Xₐ inhibierende Eigenschaften und können daher zur Bekämpfung und Verhütung von thromboembolischen Erkrankungen, wie Thrombose, myocardialem Infarkt, Arteriosklerose, Entzündungen, Apoplexie, Angina pectoris, Restenose nach Angioplastie und Claudicatio intermittens eingesetzt werden.

Die erfindungsgemäßen Verbindungen der Formel I können weiterhin Inhibitoren der Gerinnungsfaktoren VIIₐ, IXₐ und Thrombin der Blutgerinnungskaskade sein.

Die Messung der Inhibierung von Thrombin kann z.B. nach der Methode von G.F. Cousins et al. in *Circulation* **1996,** *94*, 1705-1712 erfolgen.

Die Inhibierung des Faktors Xₐ durch die erfindungsgemäßen Verbindungen und die Messung der anticoagulierenden und antithrombotischen Aktivität kann nach üblichen in vitro- oder in vivo-Methoden ermittelt werden. Ein geeignetes Verfahren wird z.B. von J. Hauptmann et al. in *Thrombosis and Haemostasis,* **1990,** *63*, 220-223 beschrieben.

Die Messung der Inhibierung von Faktor Xₐ kann z.B. nach der Methode von T. Hara et al. in *Thromb. Haemostas.,* **1994,** *71*, 314-319 erfolgen.

Die Inhibierung des Faktors VIIₐ durch die erfindungsgemäßen Verbindungen und die Messung der antikoagulierenden und antithrombotischen Aktivität kann nach üblichen in vitro- oder in vivo-Methoden ermittelt werden. Ein übliches Verfahren zur Messung der Inhibierung von Faktor VIIₐ wird z.B. von H.F. Ronning et al. in *Thrombosis Research* **1996**, *84*, 73-81 beschrieben.

Die Inhibierung von Faktor IXₐ durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Chang et al. *in Journal of Biological Chemistry* **1998,** *273*, 12089-12094 beschrieben.

Die Verbindungen der Formel I können als Arzneimittelwirkstoffe in der Human- und Veterinärmedizin eingesetzt werden, insbesondere zur Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie und/oder Claudicatio intermittens. Sie können weiterhin zur Behandlung von Tumoren, Tumorerkrankungen und/oder Tumormetastasen eingesetzt werden. Ein Zusammenhang zwischen dem Tissuefaktor TF/Faktor VIIa und der Entwicklung verschiedener Krebsarten wurde von T. Taniguchi et al. in Biomed. Health Res. (2000), 41 ("Molecular Pathogenesis of Pancreatic Cancer"), 57 - 59 aufgezeigt.

Besonders bevorzugt weisen die erfindungsgemäßen Semicarbazide eine Struktur der Formel II auf. 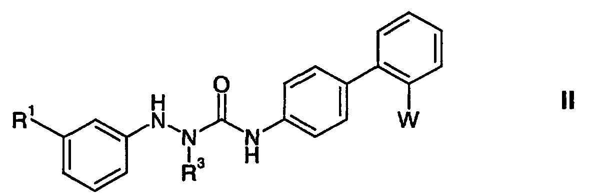 wobei R¹, R³, A und k die in Anspruch 1 angegebene Bedeutung aufweisen und W steht für S(O)ₖA, S(O)ₖNHA, CF₃, COOA, CH₂NHA, CN oder OA.

Besonders hervorzuhebende Verbindungen sind im folgenden genannt:
4'-[3-(3-Amidinophenyl)-2-propylcarbazoylamino]-biphenyl-2-sulfonamid (1),
4'-[3-(3-(N²-Hydroxyamidino)-phenyl)-2-propylcarbazoylamino]-biphenyl-2-sulfonamid (2),
4'-[3-(3-Amidinophenyl)-2-methylcarbazoylamino]-biphenyl-2-sulfonamid (3),
1-(3-Amidinophenyl)-2-methyl-4-(2'-methylsulfonylbiphenyl-4-yl)-semicarbazid (4),
4'-[3-(3-Amidinophenyl)-2-ethylcarbazoylamino]-biphenyl-2-sulfonamid (5),
1-(3-Amidinophenyl)-2-ethyl-4-(2'-methylsulfonylbiphenyl-4-yl)-semicarbazid (6),
4'-[3-(3-Amidinophenyl)-2-isopropylcarbazoylamino]-biphenyl-2-sulfonamid (7),
1-(3-Amidinophenyl)-2-isopropyl-4-(2'-methylsulfonylbiphenyl-4-yl)-semicarbazid (8),
4'-[3-(3-Amidinophenyl)-2-butylcarbazoylamino]-biphenyl-2-sulfonamid (9),
1-(3-Amidinophenyl)-2-butyl-4-(2'-methylsulfonylbiphenyl-4-yl)-semicarbazid (10),
4'-[3-(3-Amidinophenyl)-2-isobutylcarbazoylamino]-biphenyl-2-sulfonamid (11),
1-(3-Amidinophenyl)-2-isobutyl-4-(2'-methylsulfonylbiphenyl-4-yl)-semicarbazid (12),
4'-[3-(3-Amidinophenyl)-2-pentylcarbazoylamino]-biphenyl-2-sulfonamid (13),
1-(3-Amidinophenyl)-4-(2'-methylsulfonylbiphenyl-4-ylamino)-2-pentylsemicarbazid (14),
1-(3-Amidinophenyl)-4-(2'-methylsulfonylbiphenyl-4-ylamino)-2-propylsemicarbazid (15),
4'-[3-(3-Amidinophenyl)-2-(2-butyl)-carbazoylamino]-biphenyl-2-sulfonamid (16),
1-(3-Amidinophenyl)-2-(2-butyl)-4-(2'-methylsulfonylbiphenyl-4-yl)-semicarbazid(17),
4'-[3-(3-Amidinophenyl)-2-(cyclohexylmethyl)-carbazoylamino]-biphenyl-2-sulfonamid (18),
1-(3-Amidinophenyl)-2-(cyclohexylmethyl)-4-(2'-methylsulfonylbiphenyl-4-yl)-semicarbazid (19),
4'-[3-(3-Amidinophenyl)-2-benzylcarbazoylamino]-biphenyl-2-sulfonamid (20),
1-(3-Amidinophenyl)-2-benzyl-4-(2'-methylsulfonylbiphenyl-4-yl)-semicarbazid (21),
1-(3-N²-Hydroxyamidinophenyl)-2-benzyl-4-(2'-methylsulfonylbiphenyl-4-yl)-semicarbazid (22),
4'-[3-(3-Amidinophenyl)-2-phenylcarbazoylamino]-biphenyl-2-sulfonamid (23),
1-(3-Amidinophenyl)-4-(2'-methylsulfonylbiphenyl-4-yl)-2-phenylsemicarbazid (24),
N-[3-[2-Benzyl-4-(2'-methylsulfonylbiphenyl-4-yl)-semicarbazido]-phenyl-(iminomethyl)]-carbaminsäuremethylester (25),
N-[3-[2-Benzyl-4-(2'-methylsulfonylbiphenyl-4-yl)-semicarbazido]-phenyl-(iminomethyl)]-carbaminsäure-(2,2,2-trichlorethylester) (26),
N-[3-[2-Benzyl-4-(2'-methylsulfonylbiphenyl-4-yl)-semicarbazido]-phenyl-(iminomethyl)]-thiocarbaminsäure-S-ethylester (27),
N-[3-[2-Benzyl-4-(2'-methylsulfonylbiphenyl-4-yl)-semicarbazido]-phenyl-(iminomethyl)]-carbaminsäure-(4-methoxybenzylester) (28),
N-[3-[2-Benzyl-4-(2'-methylsulfonylbiphenyl-4-yl)-semicarbazido]-phenyl-(iminomethyl)]-carbaminsäureethylester (29),
N-[3-[2-Benzyl-4-(2'-methylsulfonylbiphenyl-4-yl)-semicarbazido]-phenyl-(iminomethyl)]-carbaminsäurepropylester (30),
N-[3-[2-Benzyl-4-(2'-methylsulfonylbiphenyl-4-yl)-semicarbazido]-phenyl-(iminomethyl)]-carbaminsäurebutylester (31),
N-[3-[2-Benzyl-4-(2'-methylsulfonylbipheny)-4-yl)-semicarbazido]-phenyl-(iminomethyl)]-carbaminsäureisopropylester (32),
N-[3-[2-Benzyl-4-(2'-methylsulfonylbiphenyl-4-yl)-semicarbazido]-phenyl-(iminomethyl)]-carbaminsäureisobutylester (33),
N-[3-[2-Benzyl-4-(2'-methylsulfonylbiphenyl-4-yl)-semicarbazido]-phenyl-(iminomethyl)]-carbaminsäureallylester (34),
N-[3-[2-Benzyl-4-(2'-methylsulfonylbiphenyl-4-yl)-semicarbazido]-phenyl-(iminomethyl)]-carbaminsäurephenylester (35),
N-[3-[2-Benzyl-4-(2'-methylsulfonylbiphenyl-4-yl)-semicarbazido]-phenyl-(iminomethyl)]-carbaminsäure-(2-fluorphenylester) (36),
2-Benzyl-1-[3-(N¹-(methylcarboxy)-amidino)-phenyl]-4-(2'-methylsulfonylbiphenyl-4-yl)-semicarbazid (37),
2-Benzyl-4-(2'-methylsulfonylbiphenyl-4-yl)-1-[3-(N¹-(phenylcarboxy)-amidino)-phenyl]-semicarbazid (38),
2-Benzyl-1-[3-(N¹-(isobutylcarboxy)-amidino)-phenyl]-4-(2'-methylsulfonylbiphenyl-4-yl)-semicarbazid (39),
2-Benzyl-1-[3-[N¹-(2-methylcarboxy-2-propoxycarbonyl)-amidino]-phenyl]-4-(2'-methylsulfonylbiphenyl-4-yl)-semicarbazid (40),
2-Benzyl-1-[3-[N¹-(1-(methylcarboxy)-ethoxycarbonyl)-amidino]-phenyl]-4-(2'-methylsulfonylbiphenyl-4-yl)-semicarbazid (41),
N-[3-[2-Benzyl-4-(2'-methylsulfonylbiphenyl-4-yl)-semicarbazido]-phenyl-(iminomethyl)]-carbaminsäure-(1-methyl-4-piperidinylester) (42),
N-[3-[2-Benzyl-4-(2'-methylsulfonylbiphenyl-4-yl)-semicarbazido]-phenyl-(iminomethyl)]-carbaminsäure-[2-(4-pyridyl)-ethylester] (43),
N-[3-[2-Benzyl-4-(2'-methylsulfonylbiphenyl-4-yl)-semicarbazido]-phenyl-(iminomethyl)]-carbaminsäure-(5-methyl-2-oxo-1,3-dioxol-4-ylmethylester) (44),
N-[3-[2-Benzyl-4-(2'-methylsulfonylbiphenyl-4-yl)-semicarbazido]-phenyl-(iminomethyl)]-carbaminsäure-[2-(3-pyridyl)-ethylester] (45),
N-[3-[2-Benzyl-4-(2'-methylsulfonylbiphenyl-4-yl)-semicarbazido]-phenyl-(iminomethyl)]-carbaminsäure-[2-(N,N-diethylamino)-ethylester] (46),
N-[3-[2-Benzyl-4-(2'-methylsulfonylbiphenyl-4-yl)-semicarbazido]-phenyl-(iminomethyl)]-carbaminsäure-[2-(N-morpholinyl)-ethylester] (47),
1-(3-Amidinophenyl)-2-(2-fluorbenzyl)-4-(2'-methylsulfonylbiphenyl-4-yl)-semicarbazid (48),
1-(3-Amidinophenyl)-2-(2-methylbenzyl)-4-(2'-methylsulfonylbiphenyl-4-yl)-semicarbazid (49),
1-(3-Amidinophenyl)-2-(2-chlorbenzyl)-4-(2'-methylsulfonylbiphenyl-4-yl)-semicarbazid (50),
1-(3-Amidinophenyl)-2-(3-chlorbenzyl)-4-(2'-methylsulfonylbiphenyl-4-yl)-semicarbazid (51),
4-[1-(3-Amidinophenylamino)-3-(2'-methylsulfonylbiphenyl-4-yl)-ureidomethyl]-benzoesäure (52),
1-(3-Amidinophenyl)-2-(3-methylbenzyl)-4-(2'-methylsulfonylbiphenyl-4-yl)-semicarbazid (53),
1-(3-Amidinophenyl)-4-(2'-methylsulfonylbiphenyl-4-yl)-2-(3-trifluormethylbenzyl)-semicarbazid (54),
1-(3-Amidinophenyl)-4-(2'-methylsulfonylbiphenyl-4-yl)-2-(4-trifluormethylbenzyl)-semicarbazid (55),
1-(3-Amidinophenyl)-4-(2'-methylsulfonylbiphenyl-4-yl)-2-(3-trifluormethoxybenzyl)-semicarbazid (56),
1-(3-Amidinophenyl)-2-benzyl-4-(2'-methylsulfonyl-3-fluor-biphenyl-4-yl)-semicarbazid (57),
1-(3-Amidinophenyl)-2-(3-trifluormethylbenzyl)-4-(2'methylsulfonyl-3-fluor-biphenyl-4-yl)-semicarbazid (58),
1-(3-Amidinophenyl)-2-(4-trifluormethylbenzyl)-4-(2'methylsulfonyl-3-fluor-biphenyl-4-yl)-semicarbazid (59),
1-(3-Amidinophenyl)-2-(2-trifluormethylbenzyl)-4-(2'-methylsulfonylbiphenyl-4-yl)-semicarbazid (60),
1-(3-Amidinophenyl)-2-(2-trifluormethylbenzyl)-4-(2'-methylsulfonyl-3-fluor-biphenyl-4-yl)-semicarbazid (61),
1-(3-Amidinophenyl)-2-benzyl-4-(2'-methylsulfonyl-3,5-difluor-biphenyl-4-yl)-semicarbazid (62),
1-(3-Amidinophenyl)-2-(2-trifluormethylbenzyl)-4-(2'-methylsulfonyl-3,5-difiuor-biphenyl-4-yl)-semicarbazid (63),
1-(3-Amidinophenyl)-2-(3-trifluormethylbenzyl)-4-(2'-methylsulfonyl-3,5-difluor-biphenyl-4-yl)-semicarbazid (64),
1-(3-Amidinophenyl)-2-(4-trifluormethylbenzyl)-4-(2'-methylsulfonyl-3,5-difluor-biphenyl-4-yl)-semicarbazid (65),
2-[1-(3-Amidinophenylamino)-3-(2'-sulfamoylbiphenyl-4-yl)-ureido]-essigsäureethylester (66),
3-[1-(3-Amidinophenylamino)-3-(2'-sulfamoylbiphenyl-4-yl)-ureido]-propionsäureethylester (67),
4'-[3-(3-Amidinophenyl)-2-[2-(1-methyltetrazol-5-yl)-ethyl]-carbazoylamino]-biphenyl-2-sulfonamid (68),
4'-[3-(3-Amidinophenyl)-2-(2-methoxyethyl)-carbazoylamino]-biphenyl-2-sulfonamid (69),
4'-[3-(3-Amidinophenyl)-2-(methoxymethyl)-carbazoylamino]-biphenyl-2-sulfonamid (70),
4'-[3-(3-Amidinophenyl)-2-(4-methoxybutyl)-carbazoylamino]-biphenyl-2-sulfonamid (71),
1-(3-Aminomethylphenyl)-2-benzyl-4-(2'-methylsulfonylbiphenyl-4-yl)-semicarbazid (72),
1-(3-Aminomethylphenyl)-4-(2'-methylsulfonylbiphenyl-4-yl)-2-(2-trifluormethylbenzyl)-semicarbazid (73),
1-(3-Aminomethylphenyl)-4-(2'-methylsulfonylbiphenyl-4-yl)-2-(3-trifluormethylbenzyl)-semicarbazid (74),
1-(3-Aminomethylphenyl)-4-(2'-methylsulfonylbiphenyl-4-yl)-2-(4-trifluormethylbenzyl)-semicarbazid (75),
1-(3-Aminomethylphenyl)-2-benzyl-4-(3-fluor-2'-methylsulfonylbiphenyl-4-yl)-semicarbazid (76),
1-(3-Aminomethylphenyl)-4-(3-fluor-2'-methylsulfonylbiphenyl-4-yl)-2-(2-trifluormethylbenzyl)-semicarbazid (77),
1-(3-Aminomethylphenyl)-4-(3-fluor-2'-methylsulfonylbiphenyl-4-yl)-2-(3-trifluormethylbenzyl)-semicarbazid (78),
1-(3-Aminomethylphenyl)-4-(3-fluor-2'-methylsulfonylbiphenyl-4-yl)-2-(4-trifluormethylbenzyl)-semicarbazid (79),
1-(3-Aminomethylphenyl)-2-(3-trifluormethylbenzyl)-4-(2'-methylsulfonylbiphenyl-4-yl)-semicarbazid (80),
1-(3-Aminomethylphenyl)-2-(4-trifluormethylbenzyl)-4-(2'-methylsulfonylbiphenyl-4-yl)-semicarbazid (81),
1-(3-Aminomethylphenyl)-2-benzyl-4-(2'methylsulfonyl-3-fluor-biphenyl-4-yl)-semicarbazid (82),
1-(3-Aminomethylphenyl)-2-(3-trifluormethylbenzyl)-4-(2'-methylsulfonyl-3-fluor-biphenyl-4-yl)-semicarbazid (83),
1-(3-Aminomethylphenyl)-2-benzyl-4-(2'-methylsulfonylbiphenyl-4-yl)-semicarbazid (84),
1-(3-Aminomethylphenyl)-2-(2-trifluormethylbenzyl)-4-(2'-methylsulfonyl-3-fluor-biphenyl-4-yl)-semicarbazid (85),
1-(3-Aminomethylphenyl)-2-benzyl-4-(2'-methylsulfonyl-3,5-difluor-biphenyl-4-yl)-semicarbazid (86),
1-(3-Aminomethylphenyl)-2-(2-trifluormethylbenzyl)-4-(2'-methylsulfonyl-3,5-difluor-biphenyl-4-yl)-semicarbazid (87),
1-(3-Aminomethylphenyl)-2-(4-trifluormethylbenzyl)-4-(2'-methylsutfonyl-3,5-difluor-biphenyl-4-yl)-semicarbazid (88),
2-Benzyl-1-[3-(N¹-methoxy)-amidino)-phenyl]-4-(2'-methylsulfonylbiphenyl-4-yl)-semicarbazid (89),
2-Benzyl-1-[3-(N¹-ethoxy)-amidino)-phenyl]-4-(2'-methylsulfonylbiphenyl-4-yl)-semicarbazid (90),
2-Benzyl-1-[3-(N¹-vinyloxy)-amidino)-phenyl]-4-(2'-methylsulfonylbiphenyl-4-yl)-semicarbazid (91),
2-Benzyl-1-[3-(N¹-benzyloxy)-amidino)-phenyl]-4-(2'-methylsulfonylbiphenyl-4-yl)-semicarbazid (92),
2-Benzyl-1-[3-(N¹-isopropoxy)-amidino)-phenyl]-4-(2'-methylsulfonylbiphenyl-4-yl)-semicarbazid (93),
1-[3-(N-Hydroxyamidino)-phenyl]-2-(3 trifluormethylbenzyl)-4-(2'-methylsulfonylbiphenyl-4-yl)-semicarbazid (94),
1-[3-(N-Hydroxyamidino)-phenyl]-2-(4-trifluormethylbenzyl)-4-(2'-methylsulfonylbiphenyl-4-yl)-semicarbazid (95),
1-[3-(N-Hydroxyamidino)-phenyl]-2-benzyl-4-(2'-methylsulfonyl-3-fluorbiphenyl-4-yl)-semicarbazid (96),
1-[3-(N-Hydroxyamidino)-phenyl]-2-(3-trifluormethylbenzyl)-4-(2'-methylsulfonyl-3-fluorbiphenyl-4-yl)-semicarbazid (97),
1-[3-(N-Hydroxyamidino)-phenyl]-2-(4-trifluormethylbenzyl)-4-(2'-methylsulfonyl-3-fluorbiphenyl-4-yl)-semicarbazid (98),
1-[3-(N-Hydroxyamidino)-phenyl]-2-(2-trifluormethylbenzyl)-4-(2'-methylsulfonylbiphenyl-4-yl)-semicarbazid (99),
1-[3-(N-Hydroxyamidino)-phenyl]-2-(2-trifluormethylbenzyl)-4-(2'-methylsulfonyl-3-fluorbiphenyl-4-yl)-semicarbazid (100),
1-[3-(N-Hydroxyamidino)-phenyl]2-benzyl-4-(2'-methylsulfonyl-3,5-difluorbiphenyl-4-yl)-semicarbazid (101),
1-[3-(N-Hydroxyamidino)-phenyl]-2-(2-trifluormethylbenzyl)-4-(2'-methylsulfonyl-3,5-difluor-biphenyl-4-yl)-semicarbazid (102),
1-[3-(N-Hydroxyamidino)-phenyl]-2-(3-trifluormethylbenzyl)-4-(2-methylsulfonyl-3,5-difluor-biphenyl-4-yl)-semicarbazid (103),
1-[3-(N-Hydroxyamidino)-phenyl]-2-(4-trifluormethylbenzyl)-4-(2-methylsulfonyl-3,5-difluor-biphenyl-4-yl)-semicarbazid (104).

Im weiteren sind besondere Ausführungsformen der Verbindungen der Formel I aufgeführt, wobei in den Tabellen jeweils eine verallgemeinerte Form der Gruppe von Verbindungen angegeben ist.

Die in den Formeln angegebenene Koeffizienten entsprechen den oben angegebenen Bedeutungen. Bei den zu den einzelnen Verbindungsgruppen synthetisierten Beispielen sind soweit verfügbar jeweils die gemessenen FAB-Werte angegeben.

In Tabelle 1 sind Beispiele aufgeführt für Verbindungen, in denen verschiedene Alkylreste R³ in das Molekülgerüst eingeführt sind. Im Diphenylteil wurden durch Variation der Gruppe Y, die für eine Amino- oder eine Methylgruppe steht, die Sulfonamidderivate und die Methylsulfonylderivate hergestellt.

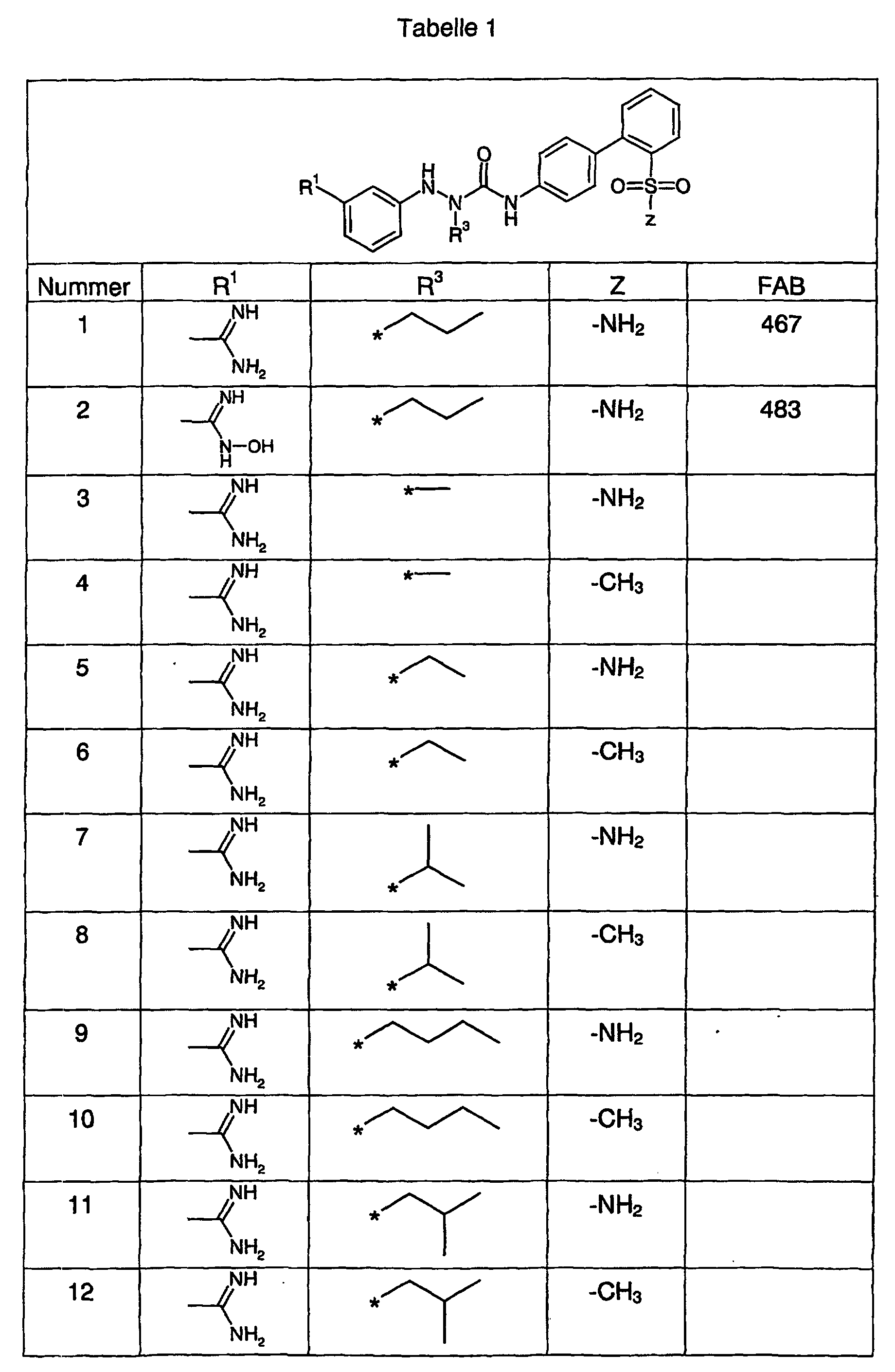

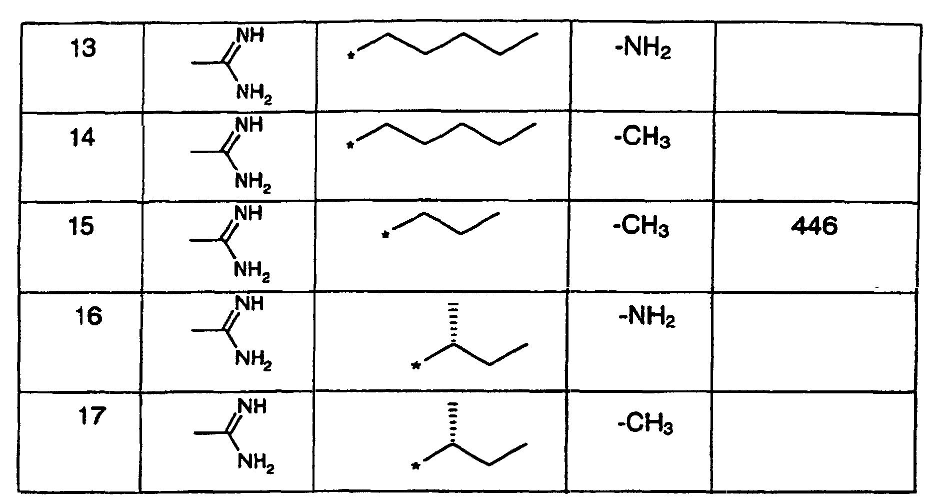

Beispiele, in denen der Rest R³ als Cycloalkylrest oder als aromatischer Rest ausgeführt ist, sind in Tabelle 2 aufgeführt. Auch hier wurden durch Variation der Gruppe Z Sulfonamidderivate sowie Methylsulfonylderivate hergestellt.

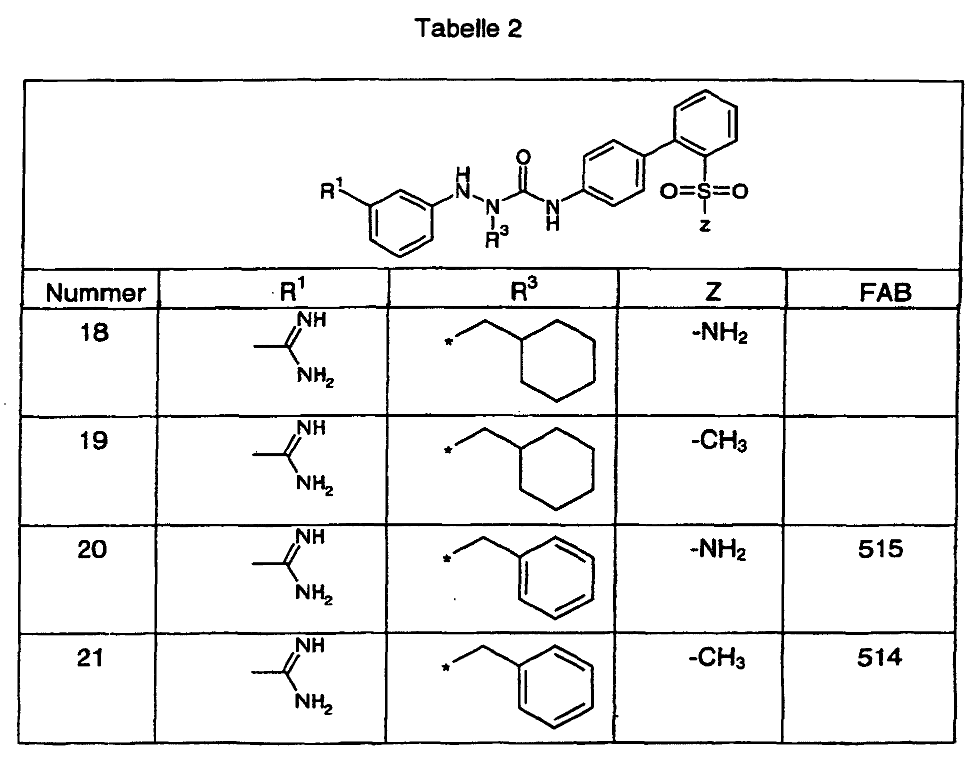

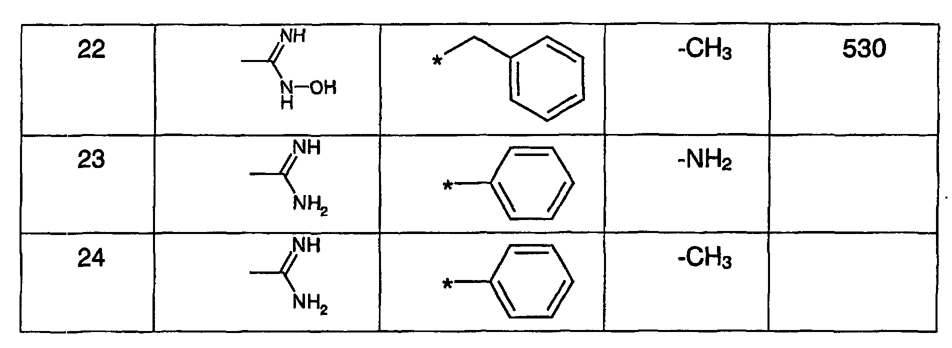

Die Verbindungen der Formel I können auch als Prodrug ausgeführt sein.
Nach der Aufnahme in den Blutkreislauf werden die Verbindungen enzymatisch gespalten und der Wirkstoff freigesetzt. In Tabelle 3 sind anhand einer aktiven Verbindung verschiedene Möglichkeiten für Prodrugs dargestellt.
Durch die Variation des Restes R¹ läßt sich beispielsweise die Aufnahmegeschwindigkeit und die Geschwindigkeit der Freisetzung des Wirkstoffes beeinflussen. Die in Tabelle 3 angegebenene Beispiele für die Gruppe R¹ lassen sich ohne weiteres auf andere Wirkstoffe der Formel I übertragen, wie sie beispielsweise in den Tabellen 1 und 2 dargestellt sind.

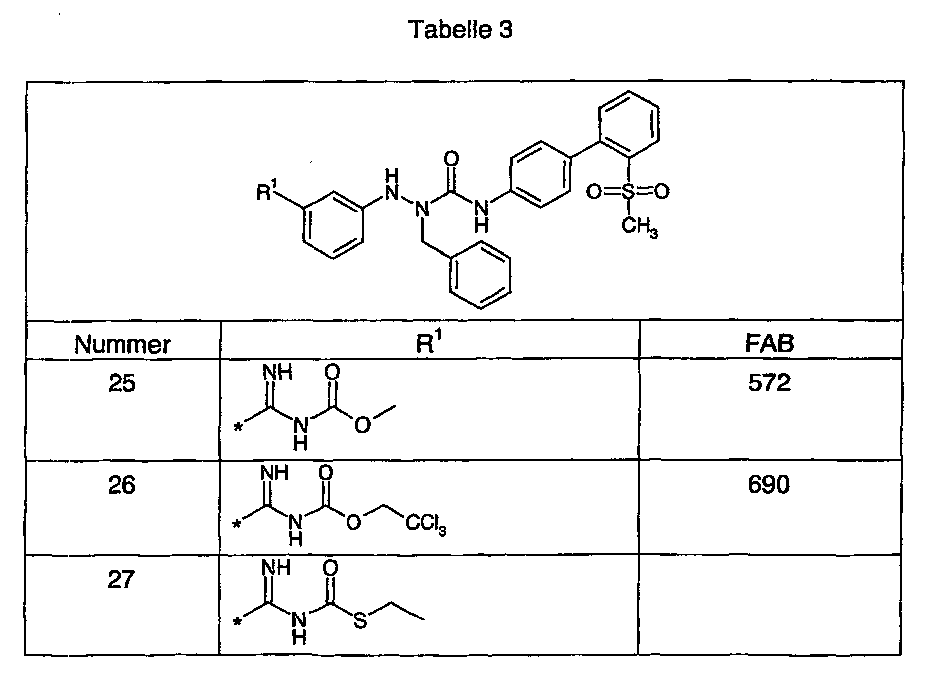

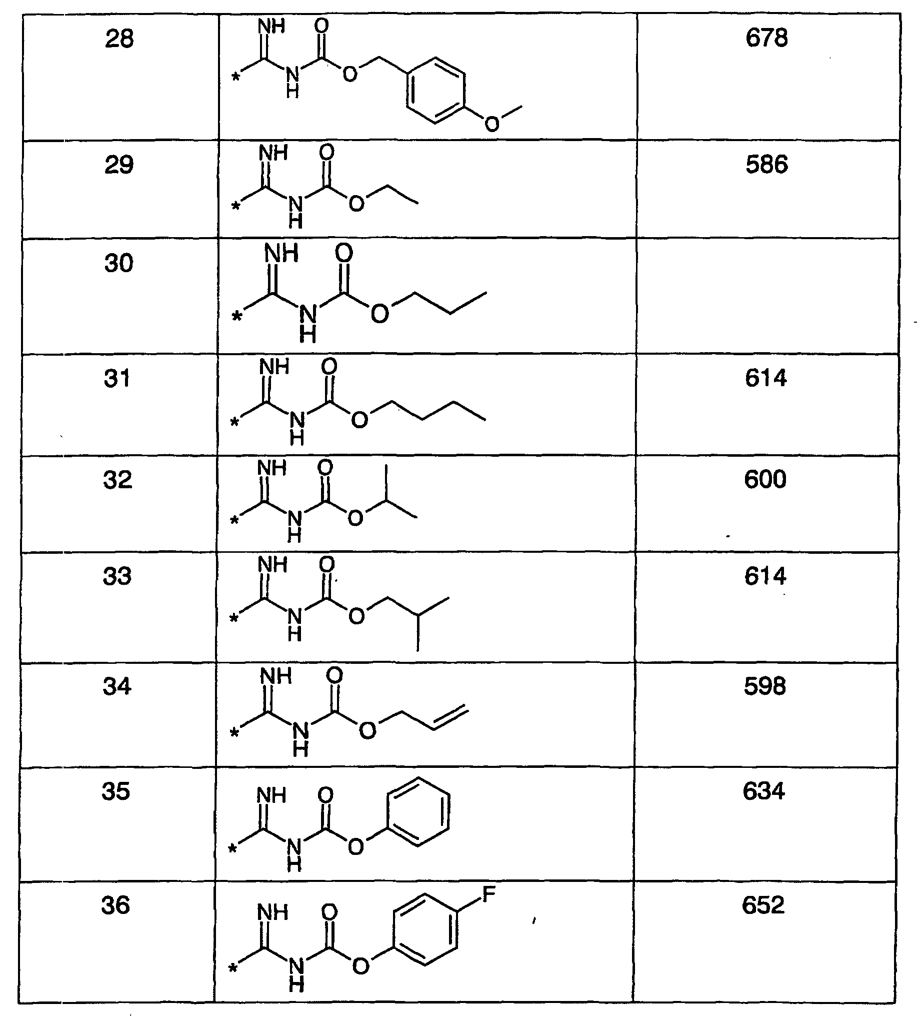

Weitere Beispiele für eine Variation des Restes R¹ für eine Herstellung von Prodrugs sind in Tabelle 4 angegeben. Die Herstellung der in Tabelle 3 und 4 dargestellten Prodrugs erfolgt analog der Vorschriften, welche beschrieben sind in S.N. Rahamthullah et al J. Med. Chem. 1999, 42, 3994 - 4000.

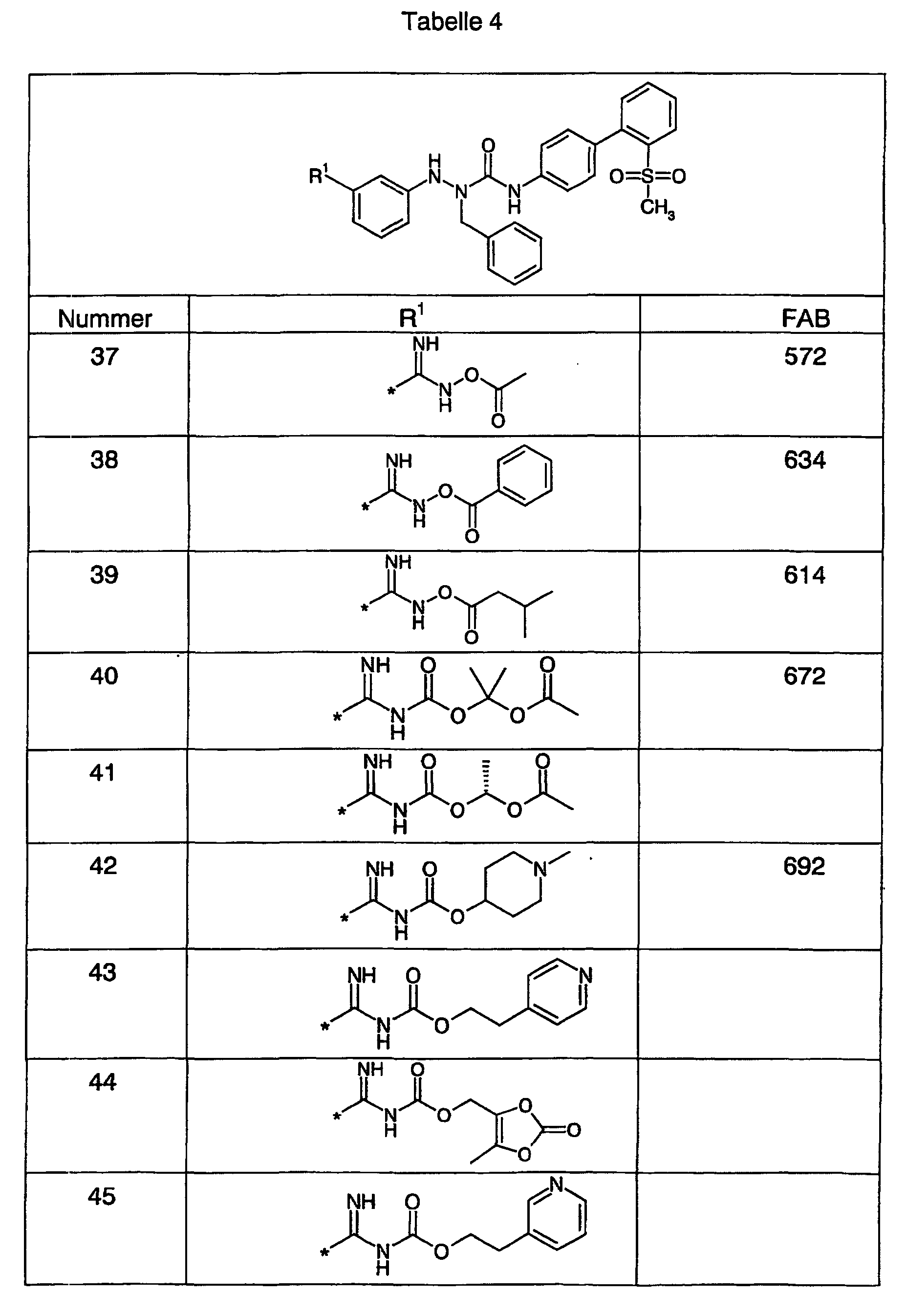

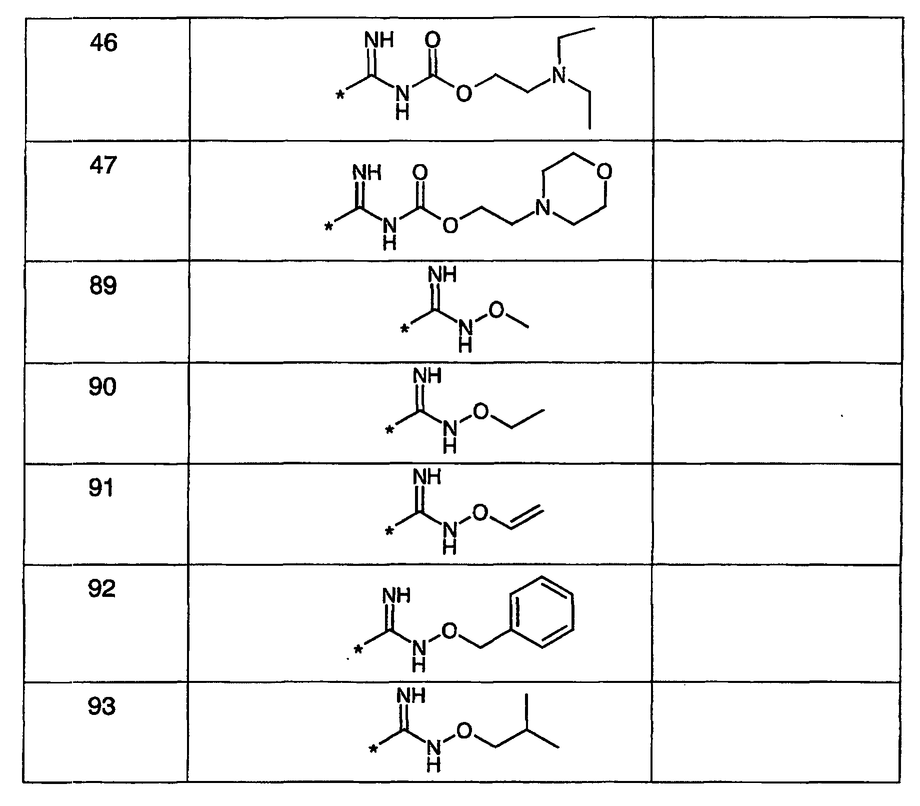

Durch Substitution der als Rest R³ eingeführten Alkyl- oder Arylgruppen mit z.B. Halogenatomen, Alkylgruppen oder Carboxylgruppen läßt sich die Polarität des Moleküls beeinflussen. Durch die Einführung von Fluoratomen wird die Verbindung lipophiler und wird daher leichter resorbiert. Beispiele für derartige Verbindungen sind in Tabelle 5 aufgeführt. Dabei wurde für einen bestimmten Wirkstoff die Gruppe R³ variiert. Eine derartige Variation kann ohne weiteres auch für andere Reste R¹ und R⁴ durchgeführt werden.

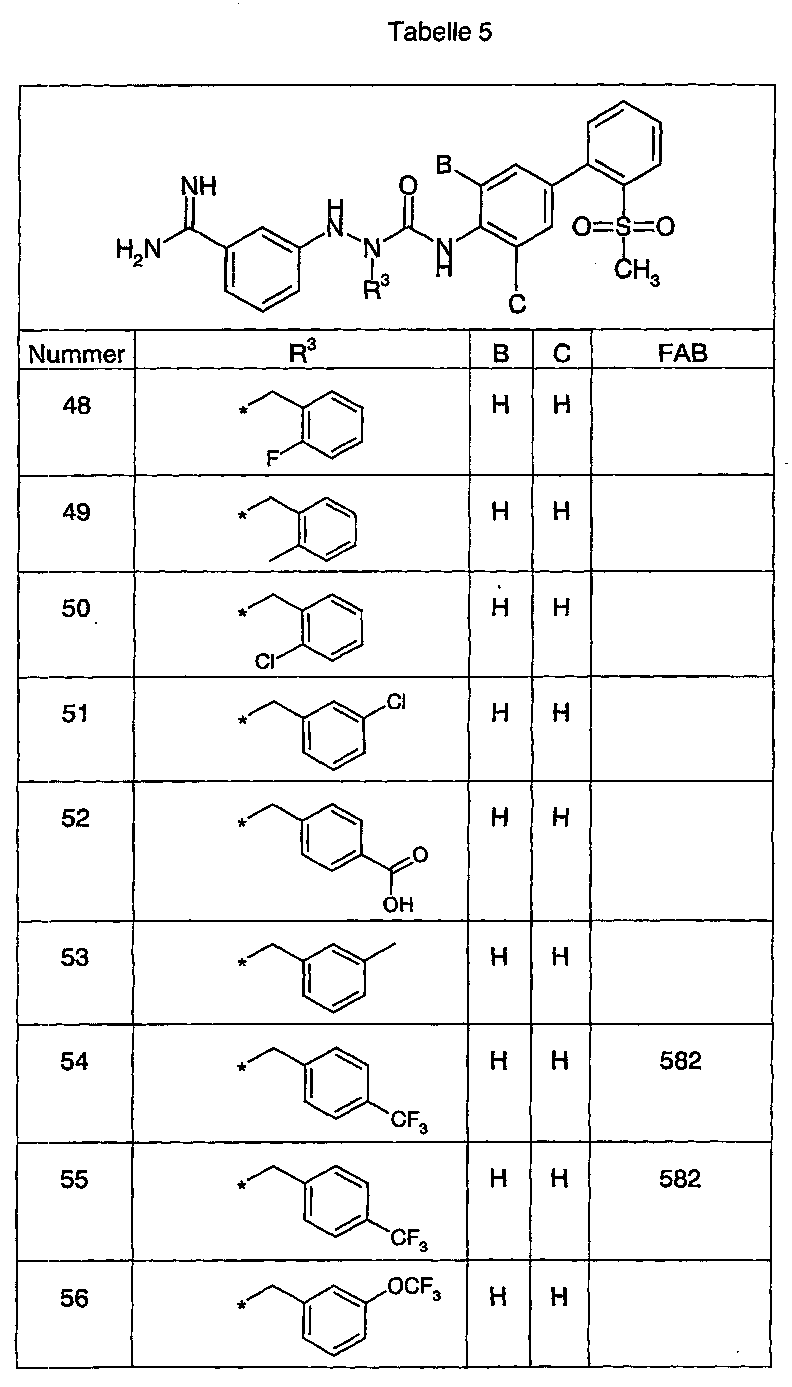

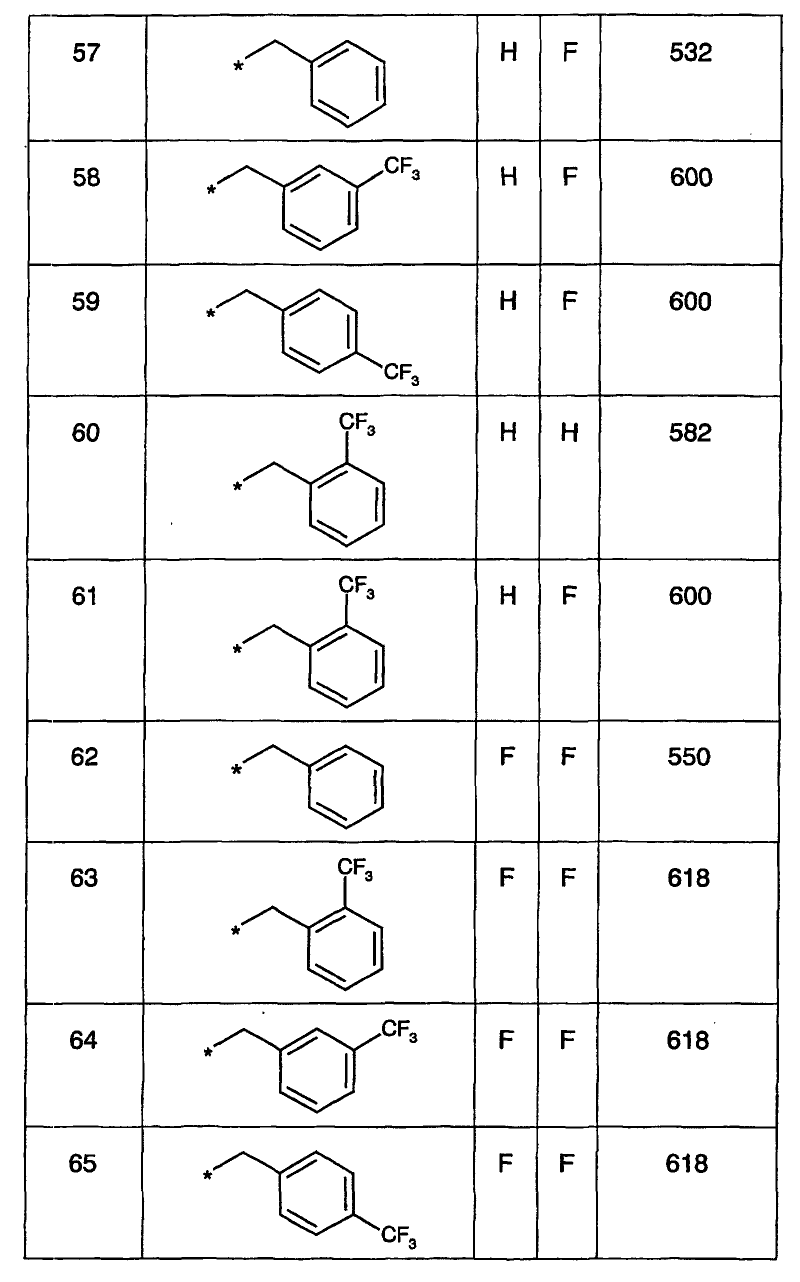

Weitere Beispiele für die Reste R³, welche Heteroatome enthalten, sind in Tabelle 6 aufgeführt. Die Variation des Restes R³ wurde dabei beispielhaft an einem Sulfonamidderivat vorgenommen.

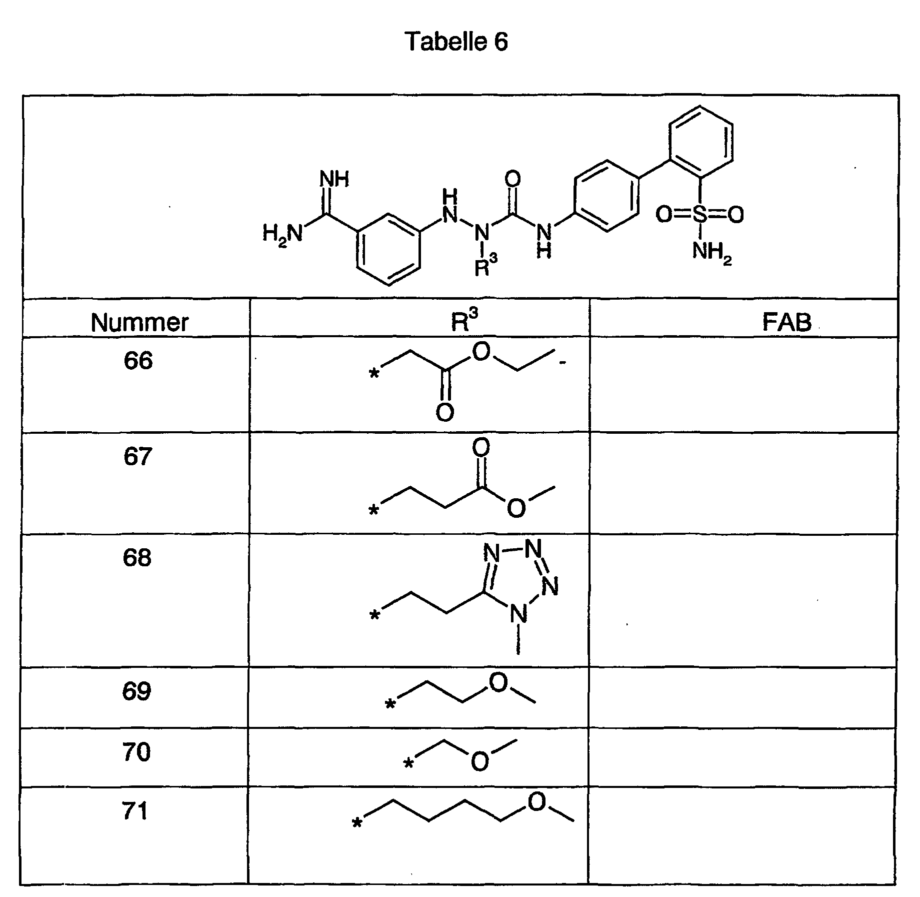

Beispiele, in denen die Lipophilie der Verbindungen variiert wurde, sind in Tabelle 7 aufgeführt. Dabei umfaßt bei einem Teil der Verbindungen der Rest R³ ein Trifluormethylgruppe.
Ferner wurden bei einem Teil der Verbindungen im Biphenylteil des Moleküls ein oder zwei Fluoratome eingeführt. Die Variationen lassen sich ohne weiteres auf die anderen Verbindungen der Formel I übertragen.

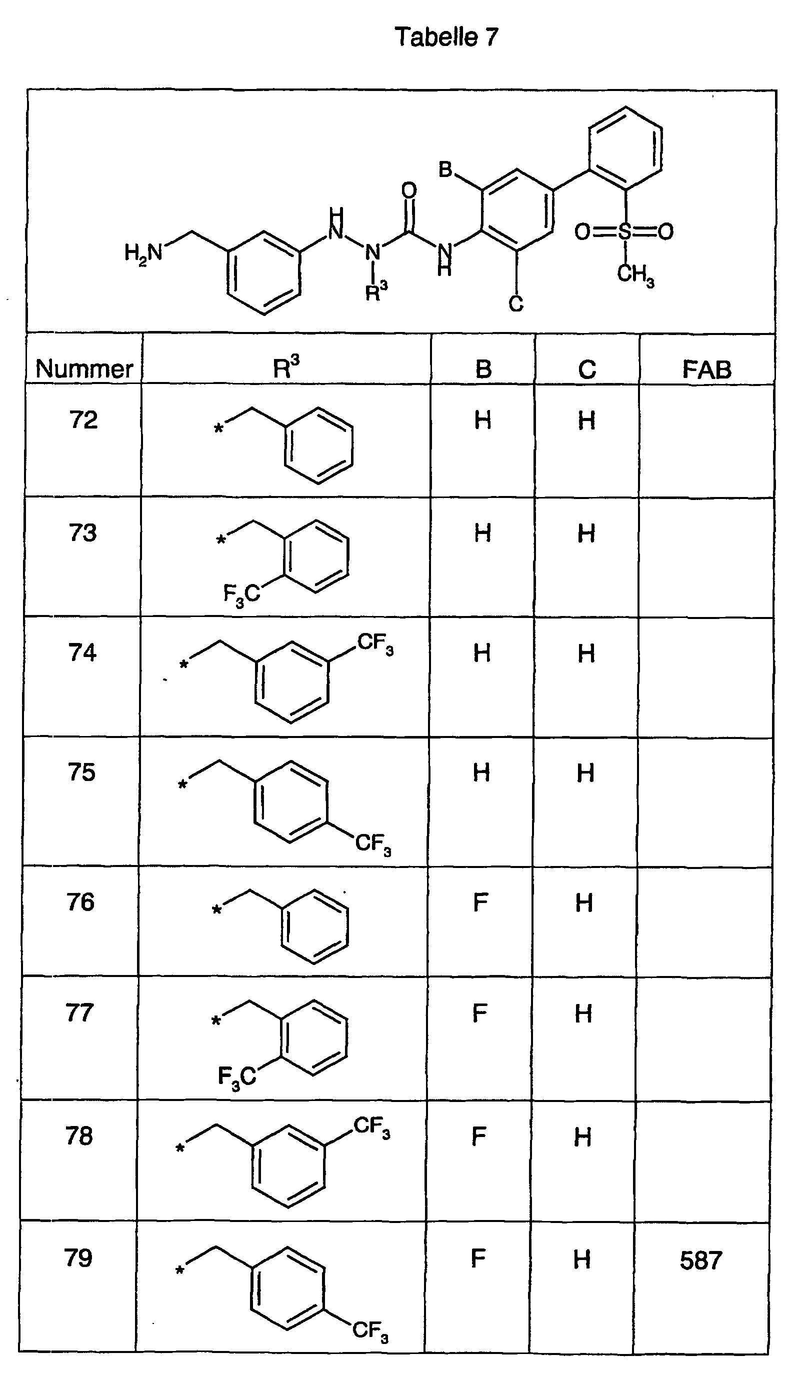

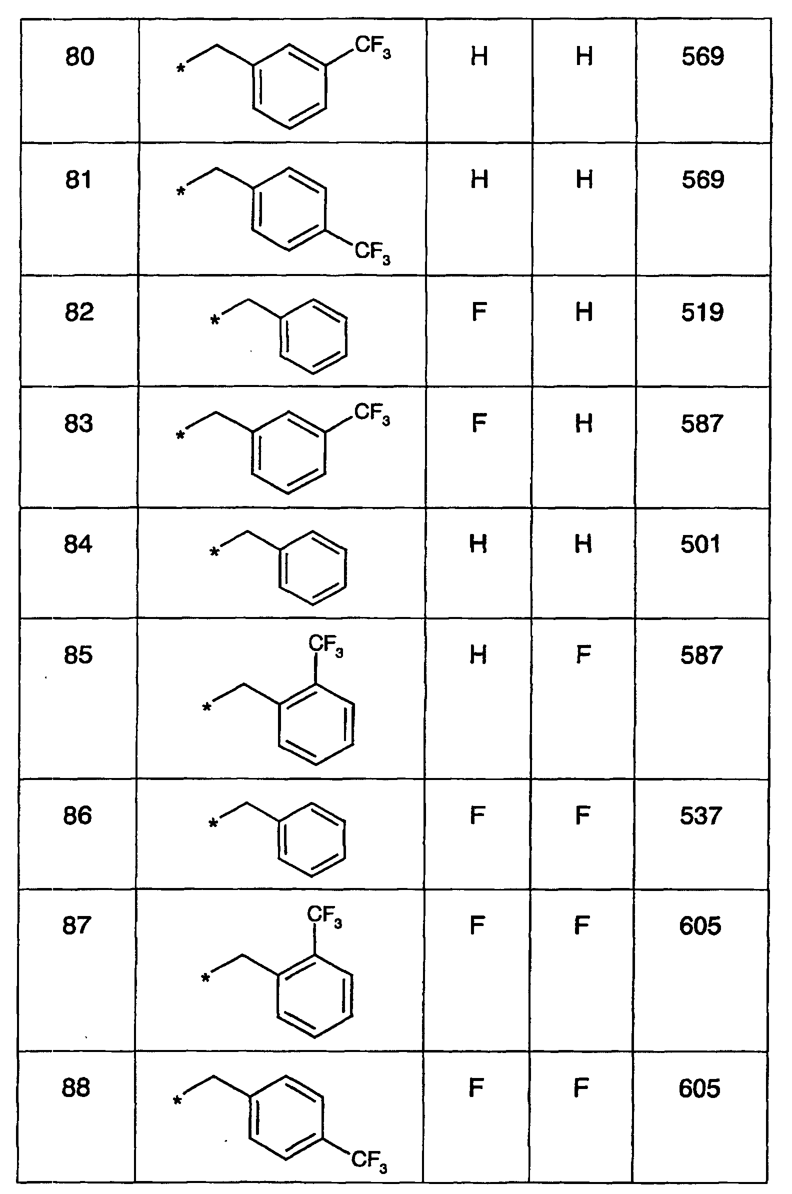

Weitere Verbindungen sind in Tabelle 8 aufgeführt.

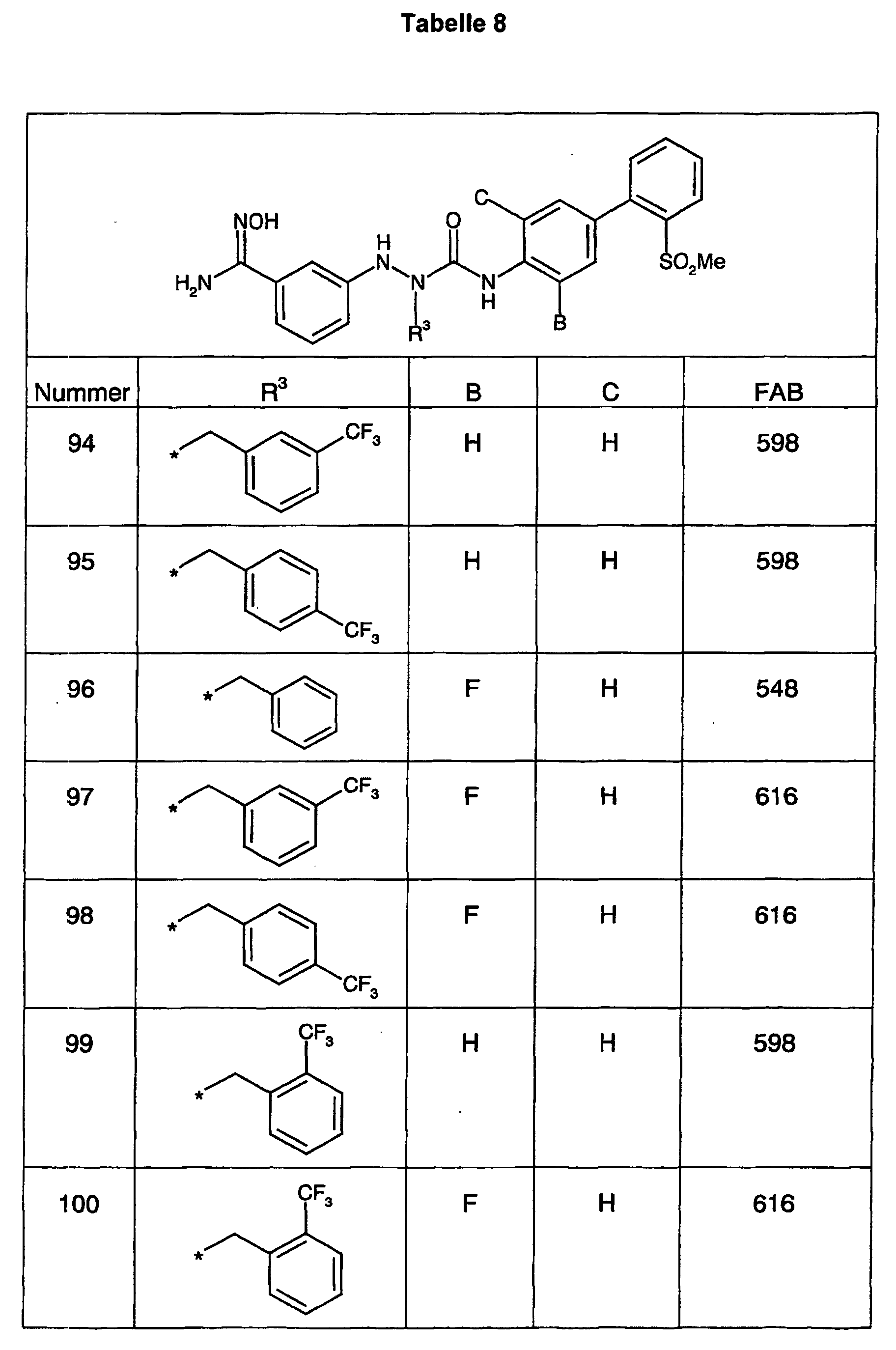

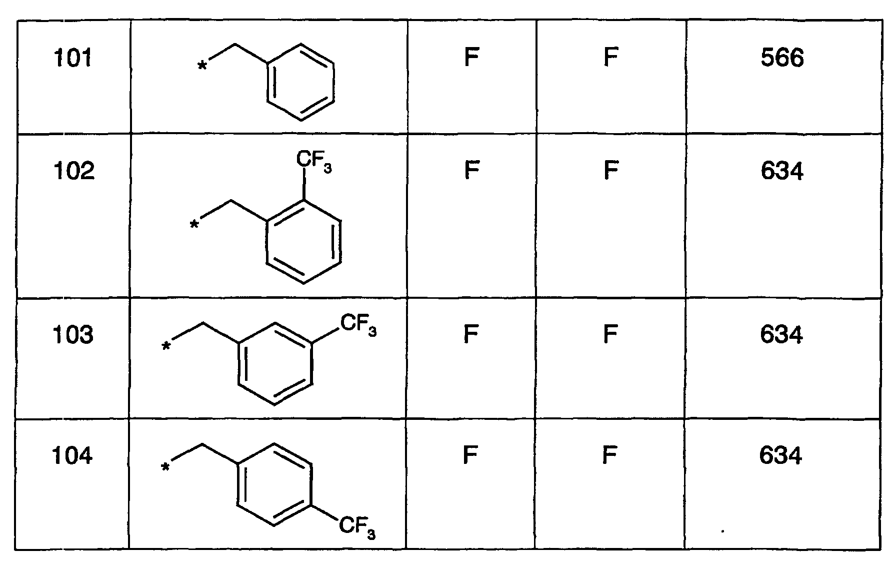

Die oben anhand ausgewählter Verbindungen dargestellten Strukturelemente der erfindungsgemäßen Verbindungen lassen sich beliebig kombinieren. Dadurch lassen sich die Verbindungen auf die beabsichtigte therapeutische Anwendung abstimmen.

Die Verbindungen der Formel I lassen sich nach an sich bekannten Verfahren herstellen. Einige beispielhafte Synthesewege werden im weiteren vorgestellt.

Der Aufbau des Molekülgerüstes wird anhand von Schema 1 erklärt.

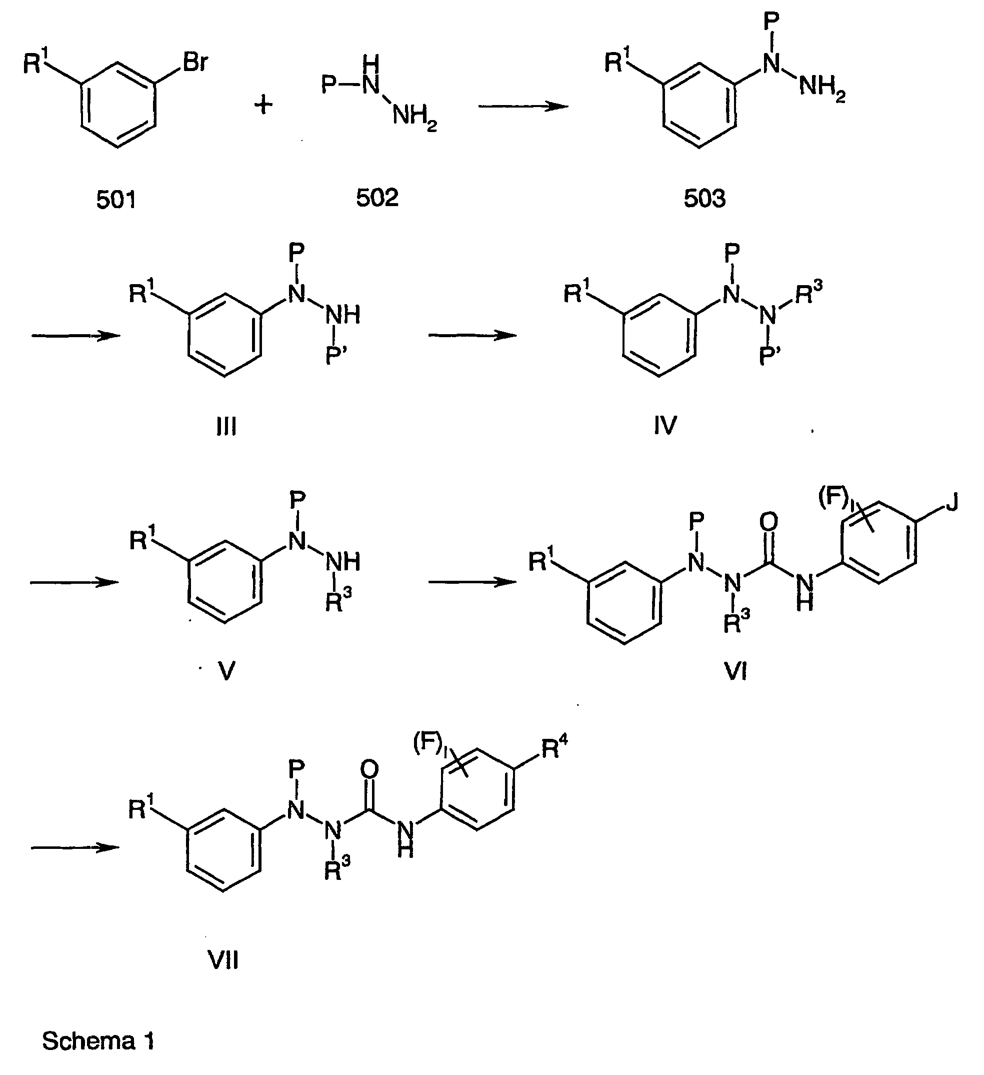

Die Synthese geht aus vom Brombenzolderivat 501. Der Rest R¹ ist bevorzugt eine Gruppe, die zu einem späteren Zeitpunkt der Synthese in eine Amidinogruppe überführt werden kann. Entsprechende Strukturelemente werden weiter unten vorgestellt. Das Brom bildet die Abgangsgruppe für die Reaktion mit dem geschütztem Hydrazinderivat 502 (Wang Z. et al. Tetrahedron Lett. 1999, 40, 3543 - 3546). Das Bromatom kann daher auch durch andere geeignete Abgangsgruppen, beispielsweise andere Halogenide oder Sulfonate ersetzt werden. Das Hydrazinderivat 502 weist eine Schutzgruppe P auf. Als Schutzgruppe können übliche Schutzgruppen für Aminogruppen verwendet werden. Als Schutzgruppe besonders geeignet ist die BOC-Schutzgruppe. Durch Umsetzung der Verbindungen 501 und 502 wird das geschützte Phenylhydrazinderivat 503 erhalten. Die freie NH₂-Gruppe wird im nächsten Reaktionsschritt durch die Schutzgruppe P' geschützt. Man erhält die Zwischenverbindung III. Die Schutzgruppen P u. P' werden nach Möglichkeit unterschiedlich gewählt, um eine selektive Entschützung der beiden Stickstoffatome zu ermöglichen. Als Schutzgruppe P' können, wie oben erläutert, die dem Fachmann bekannten Schutzgruppen für Aminogruppen verwendet werden. Als nächstes erfolgt die Einführung des Restes R³ indem beispielsweise die Verbindung III mit einem Alkylhalogenid zur Verbindung IV umgesetzt wird. Verbindung IV wird nun selektiv am terminalen Stickstoff entschützt unter Erhalt der Verbindung V. Durch Umsetzung mit gegebenenfalls F-substituiertem Jodphenylisocyanat gelangt man zur Verbindung VI. Das Jodatom in der Verbindung VI läßt sich substituieren wodurch der Rest R⁴ in das Molekül eingeführt werden kann. Man erhält Verbindung VII. Der Rest R⁴ liegt dabei in geeignet geschützter Form vor. Beispiele werden weiter unten erläutert. Die Verbindung VII enthält mit den Resten R¹, P und R⁴ im allgemeinen noch geschützte Strukturelemente. Die Abspaltung der Schutzgruppe P erfolgt nach üblichen Verfahren, beispielsweise mit Säure.

Schema 2 zeigt geeignete Reste R¹, die bei dem oben vorgestellten Reaktionsschritten unverändert bleiben und dann ausgehend von Verbindung VII freigesetzt werden können. Aus Gründen der Übersichtlichkeit ist in Schema 2 nur die Phenylgruppe mit dem Rest R¹ dargestellt.

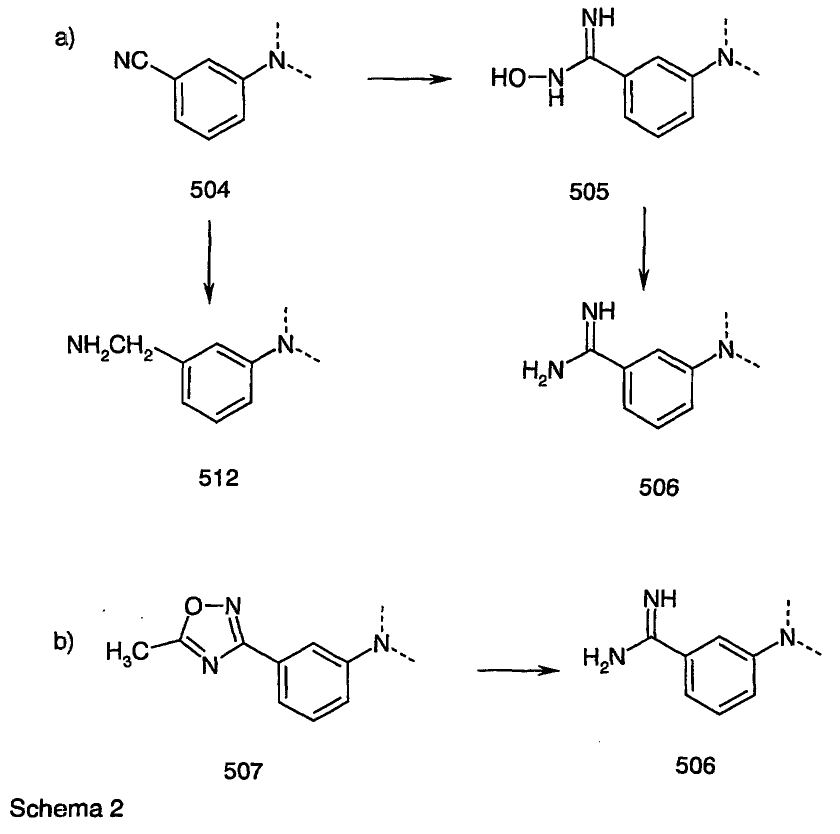

Die erste Möglichkeit besteht in der Einführung einer Nitrilgruppe. Diese läßt sich mit Hydroxylammoniumchlorid und Triethylamin in die Hydroxyamindino-Gruppe (505) überführen. Diese Verbindungen können bereits als Pro drug verwendet werden. Durch Reduktion mit Raney-Nickel unter Wasserstoffatmosphäre wird die Amidino-Gruppe (506) freigesetzt. Ebenfalls durch Reduktion entsteht aus dem Nitril (504) das Aminomethylderivat (512).

Eine weitere Möglichkeit ist unter b) gezeigt. Diese Gruppe (507) läßt sich ebenfalls mit Raney-Nickel und Wasserstoff in die Amidino-Gruppe (506) überführen.

In Schema 3 sind Möglichkeiten dargestellt, welche die Einführung einer Sulfonamid-Gruppe (a) sowie einer Methylsulfonyl-Gruppe (b) erlauben. Auch hier ist aus Gründen der Übersichtlichkeit nur der wesentliche Molkülteil (R³) dargestellt.

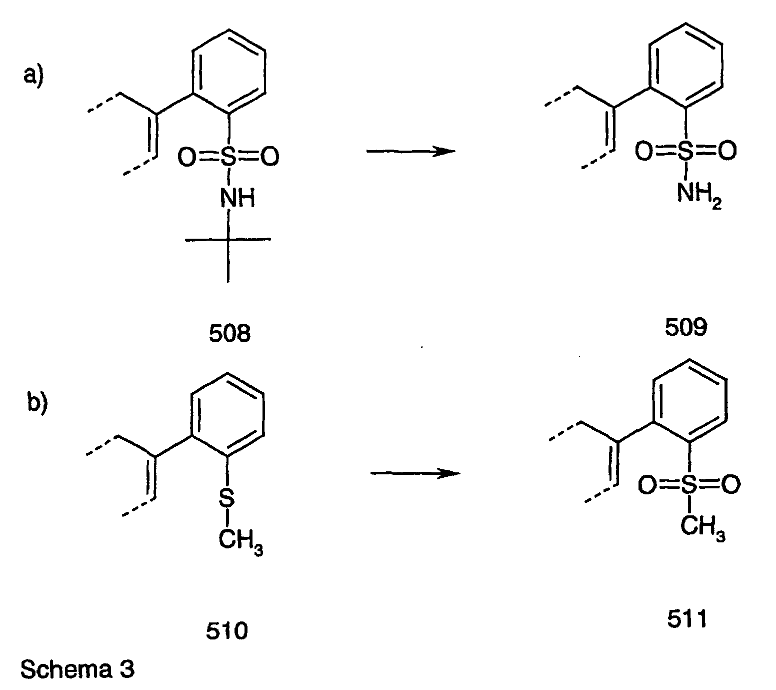

Für die Herstellung von Sulfonamiden wird die Amino-Gruppe geeignet geschützt. Unter (a) ist der Stickstoff zum Beispiel mit einer tert-Butylgruppe geschützt. Die Abspaltung der Gruppe erfolgt mit Säure z.B. Trifluoressigsäure.

Zur Einführung der Methylsulfonyl-Gruppe wird von der entsprechenden Methylthio-Verbindung (510) ausgegangen. Ausgehend von der entsprechenden Verbindung VII wird die Methylthio-Gruppe z.B. mit Natriumperborat-Trihydrat in Essigsäure zur Methylsulfonyl-Verbindung 511 oxidiert.

Die o.a. Reaktionsschemata können vom Fachmann ohne weiteres variiert werden. Z. B. können andere Abgangs- oder Schutzgruppen eingesetzt werden. Sofern racemische Gemische bei den Reaktionen erhalten werden, können aus diesen auf die übliche Weise durch Umsetzung mit einem optisch aktiven Trennmittel Diastereomere gebildet werden, die dann nach üblichen Verfahren aufgetrennt werden. Vorteilhaft ist auch eine chromatographische Enantiomerentrennung mit Hilfe eines optisch aktiven Trennmittels (z.B. Dinitrobenzylphenylglycin, Cellulosetriacetat oder andere Derivate von Kohlenhydraten oder auf Kieselgur fixierte, chiral derivatisierte Methacrylatpolymere).

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nicht chemischem Weg. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaftend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate zum Beispiel zur Herstellung von Injektionspräparaten verwendet werden. Die angegebenen Zubereitungen können sterilisiert sein und/oder Hilfsstoffe, wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder ein oder mehrere weitere Wirkstoffe enthalten, z.B. ein oder mehrere Vitamine.

Die Verbindungen der Formel I und ihre physiologisch unbedenklichen Salze können bei der Bekämpfung und Verhütung von thromboembolischen Erkrankungen wie Thrombose, myokardialem Infarkt, Arteriosklerose, Entzündungen, Apoplexie, Angina pectoris, Restenose nach Angioplastie und Claudicatio intermittens verwendet werden.

Dabei werden die erfindungsgemäßen Substanzen in der Regel vorzugsweise in Dosierungen zwischen 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von verschiedenen Faktoren ab, beispielsweise von der Wirksamkeit der eingesetzten speziellen Verbindung, vom Alter, Körpergewicht, allgemeinem Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Die Erfindung wird anhand von Beispielen näher erläutert.

### Beispiel A: Synthese von 4'-[3-(3-Amidinophenyl)-2-propylcarbazoylamino]-biphenyl-2-sulfonamid 1

Die Synthese ist in Schema 4 dargestellt.

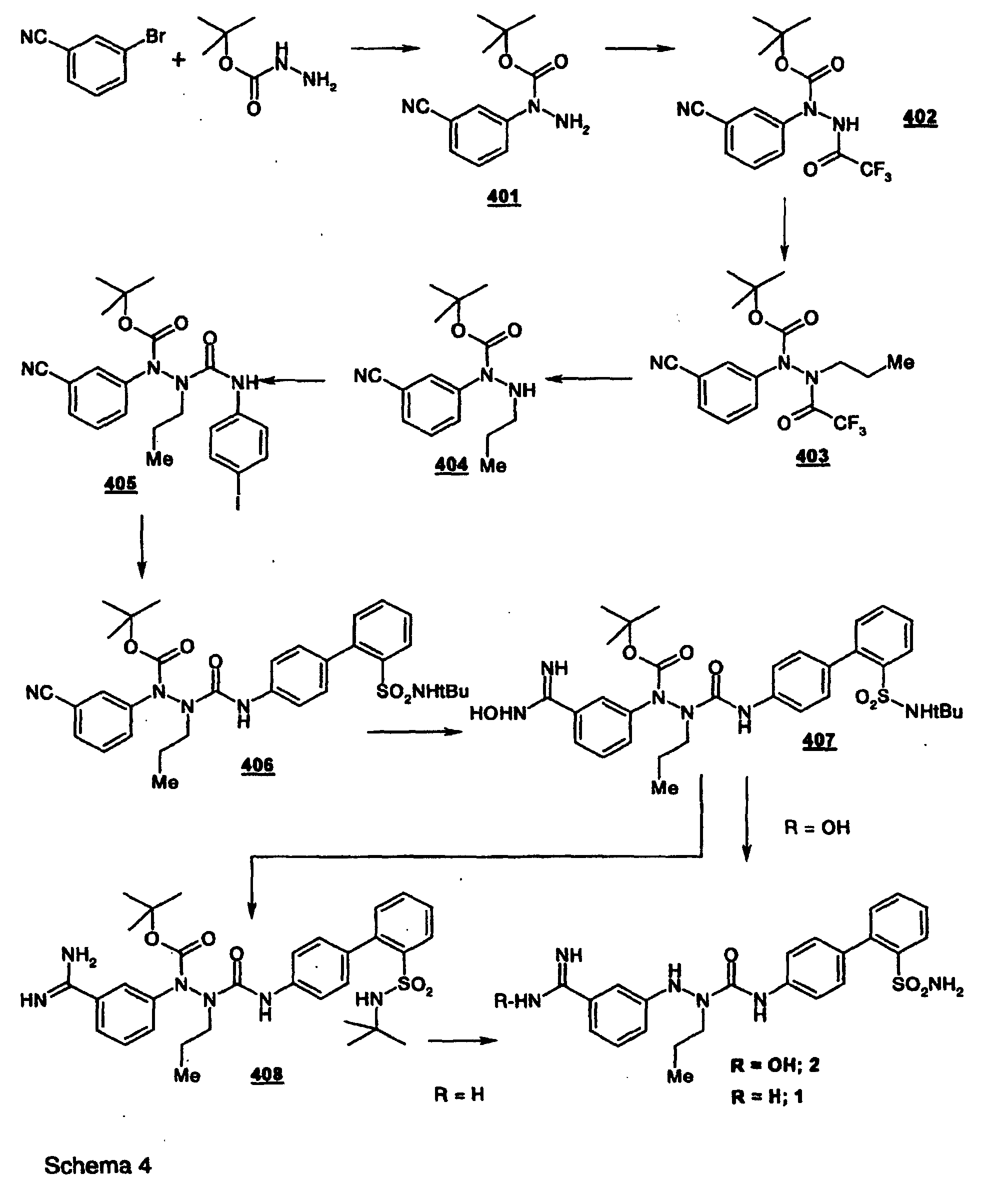

### 2-(3-Cyanophenyl)-carbazinsäure-tert-butylester 401

50,0 g (0,275 mol) 3-Brombenzonitril in 400 ml THF werden unter Stickstoffatmosphäre mit 36,345 g (0,275 mol) tert-Butylcarbazat, 89,6 g (0,275 mol) Cäsiumcarbonat, 1,581 g (0,003 mol) Bis(dibenzylidenaceton)palladium und 4,602 g (0,008 mol) 1,1'-Bis-(diphenylphosphino)-ferrocen versetzt und anschließend 18 h bei 110°C gerührt. Nach dem Abkühlen und üblicher Aufarbeitung erhält man so 15,8 g (24,7 %) **401** als Öl; MS(FAB) = 234. [Die Reaktion wird analog einer Vorschrift von Wang et al. Tetrahedron Lett. **1999,** *40*, 3543 durchgeführt].

### 2-(3-Cyanophenyl)-3-(2,2,2-trifluoracetyl-carbazinsäure-tert-butylester 402

10,7 g (46 mmol) **401** werden in 200 ml THF mit 9,54 ml (68,8 mmol) Triethylamin versetzt. Unter Kühlung auf 5°C werden 7,02 ml (50,46 mmol) Trifluoressigsäureanhydrid langsam zugetropft. Nach 6 h Rühren wird wie üblich aufgearbeitet und man erhält so 14,3 g (94,7 %) **402** als Öl, welches direkt in Stufe 3 eingesetzt wird.

### 2-(3-Cyanophenyl)-3-propyl-3-(2,2,2-trifluoracetyl)-carbazinsäure-tert-butylester 403

5,0 g (15,19 mmol) des Rohproduktes **402** werden unter Stickstoffatmosphäre in 100 ml DMF mit 7,42 g (22,77 mmol) Cäsiumcarbonat versetzt. Nach 30 min werden 3,87 ml (22,77 mmol) 1-lodpropan langsam zugetropft und anschließend 18 h bei RT gerührt. Nach üblicher Aufarbeitung erhält man so 5,64 g (100 %) **403** als Öl, welches direkt in Stufe 4 eingesetzt wird.

### 2-(3-Cyanophenyl)-3-propyl-carbazinsäure-tert-butylester 404

5,64 g (15,187 mmol) Rohprodukt **403** werden in 100 ml Methanol gelöst und bei RT mit 10 ml VE-Wasser und 1,08 g (45,24 mmol) Lithiumhydroxid versetzt. Anschließend wird das Reaktionsgemisch 5 h gerührt. Nach üblicher Aufarbeitung erhält man so 2,67 g (63,8 %) **404** als Öl; MS(FAB) = 276.

### 2-(3-Cyanophenyl)-3-(4-jodphenylaminocarbonyl)-3-propylcarbazinsäure-tert-butylester 405

2,0 g (7,273 mmol) **404** werden in 5,0 ml Pyridin mit 1,78 g (7,265 mmol) 4-lodphenylisocyanat 1,5 h bei RT gerührt. Nach üblicher Aufarbeitung erhält man so 3,2 g (84,7 %) **405** als Kristalle mit einem Schmelzpunkt von 184-185°C; MS(FAB) = 521.

### 4'-[3-tert-Butoxycarbonyl-3-(3-cyanophenyl)-2-propylcarbazoylamino]-N-tert-butylbiphenyl-2-sulfonamid 406

1,5 g (2,883 mmol) **405** und 1,112 g (4,324 mmol) 2-(t-Butylaminosulfonyl)-phenylboronsäure werden in 100 ml Ethylenglycoldimethylether gelöst, mit 63 mg (0,086 mmol) PdCl₂(dppf) und 20,0 ml 2N Natriumcarbonatlösung versetzt und anschließend 3 h bei 110°C unter Stickstoffatmosphäre gerührt. Nach üblicher Aufarbeitung erhält man so 1,32 g (75,6 %) **406** als Kristalle mit einem Schmelzpunkt von 173-174°C; MS(FAB) = 606.

### 4'-[3-tert-Butoxycarbonyl-3-[3-(N¹-hydroxyamidino)-phenyl]-2-propylcarbazoylamino]-N-tert-butylbiphenyl-2-sulfonamid 407

Eine Suspension von 1,0 g (1,651 mmol) **406** in 25,0 ml Ethanol werden mit 0,459 g (6,604 mmol) Hydroxylammoniumchlorid und 0,916 ml (6,604 mmol) Triethylamin versetzt und 5 h unter Rückfluss gerührt. Nach üblicher Aufarbeitung erhält man so 1,05 g (99,6 %) **407** als Kristalle mit einem Schmelzpunkt von 218-219°C; MS(FAB) = 639.

### 4'-[3-(3-Amidinophenyl)-3-tert-butoxycarbonyl-2-propylcarbazoylamino]-N-tert-butylbiphenyl-2-sulfonamid 408

0,7 g (1,096 mmol) **407** werden in 10,0 ml Methanol gelöst, mit 0,25 ml Essigsäure und 0,3 g Raney Nickel wasserfeucht versetzt und 18 h unter H₂-Atmosphäre gerührt. Nach üblicher Aufarbeitung erhält man so 0,62 g (90,8 %) **408** als Kristalle mit einem Schmelzpunkt >300°C; MS(FAB) = 623.

### 4'-[3-(3-(N²-Hydroxyamidino)-phenyl)-2-propylcarbazoylamino]-biphenyl-2-sulfonamid (2)

0,2 g (0,313 mmol) **407** werden in 5,0 ml Trifluoressigsäure gelöst und 18 h bei 5°C gerührt. Nach üblicher Aufarbeitung erhält man so 0,15 g (99,3 %) **2** als Kristalle mit einem Schmelzpunkt von 163-164°C; MS(FAB) = 483.

### 4'-[3-(3-Amidinophenyl)-2-propylcarbazoylamino]-biphenyl-2-sulfonamid (1)

0,5 g (0,803 mmol) **408** werden in 5,0 ml Trifluoressigsäure gelöst und 18 h bei 5°C gerührt. Nach üblicher Aufarbeitung erhält man so 0,15 g (99,3 %) **1** als Kristalle mit einem Schmelzpunkt von 215-216°C; MS(FAB) = 467.

### Beispiel B: Synthese von 1-(3-Amidinophenyl)-4-(2'-methylsulfonylbiphenyl-4-ylamino)-2-propylsemicarbazid 15

Die Synthese ist in Schema 5 dargestellt:

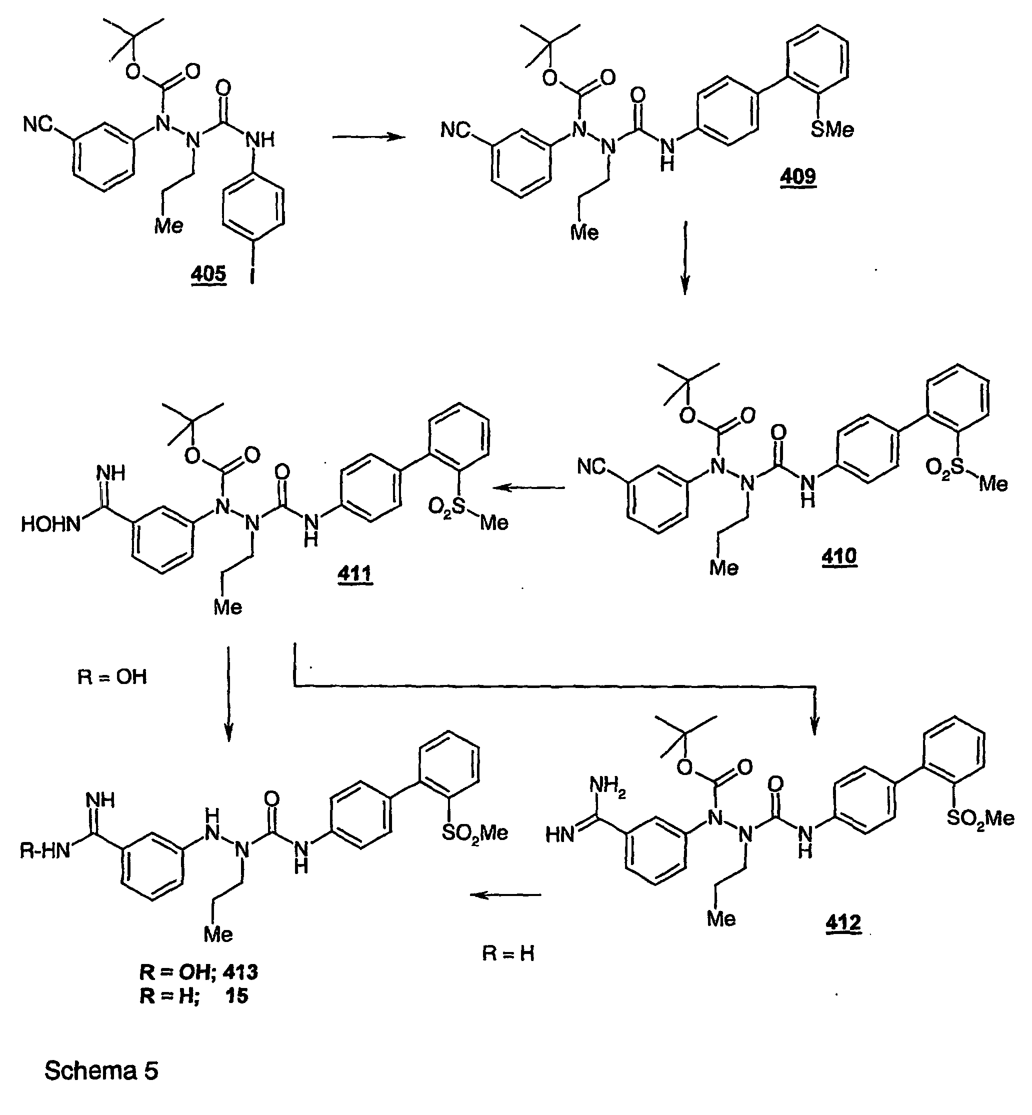

### 2-(3-Cyanophenyl)-3-(2'-methylthiobiphenyl-4-ylaminocarbonyl)-3-propylcarbazinsäure-tert-butylester 409

1,5 g (2,883 mmol) **405** und 0,968 g (5,766 mmol) 2-(Methylthio)-phenylboronsäure werden in 100 ml Ethylenglycoldimethylether gelöst, mit 63 mg (0,086 mmol) PdCl₂(dppf) und 20,0 ml 2N Natriumcarbonatlösung versetzt und anschließend 3 h bei 110°C unter Stickstoffatmosphäre gerührt. Nach üblicher Aufarbeitung erhält man so 1,28 g (75,9 %) **409** als Kristalle mit einem Schmelzpunkt von 184-185°C; MS(FAB) = 517.

### 2-(3-Cyanophenyl)-3-(2'-methylsulfonylbiphenyl-4-ylaminocarbonyl)-3-propylcarbazinsäure-tert-butylester 410

0,8 g (1,548 mmol) **409** werden mit 1,433 g (9,312 mmol) Natriumperborat-Trihydrat in 15 ml Essigsäure suspendiert und 18 h bei 60°C gerührt. Nach üblicher Aufarbeitung erhält man so 0,65 g (76,5 %) **410** als Kristalle mit einem Schmelzpunkt von 194-195°C; MS(FAB) = 549.

### 2-[3-(N¹-Hydroxyamidino)-phenyl]-3-(2'-methylsulfonylbiphenyl-4-y(aminocarbonyl)-3-propylcarbazinsäure-tert-butylester 411

Eine Lösung von 0,6 g (1,094 mmol) **410** wird in 25 ml Ethanol gelöst, mit 0,304 g (4,376 mmol) Hydroxylammoniumchlorid und 0,608 ml (4,376 mmol) Triethylamin versetzt und anschließend 18 h unter Rückfluß gerührt. Nach üblicher Aufarbeitung erhält man so 0,11 g (17,3 %) **411** als Kristalle mit einem Schmelzpunkt von 187-188°C; MS(FAB) = 582.

### 1-(3-N²-Hydroxyamidinophenyl)-4-(2'-methylsulfonylbiphenyl-4-ylamino)-2-propylsemicarbazid 413

50,0 mg (0,086 mmol) **411** werden in 1,0 ml 4N HCl in Dioxan gelöst und 18 h bei RT gerührt. Nach üblicher Aufarbeitung erhält man so 0,11 g (17,3 %) **413** als Kristalle mit einem Schmelzpunkt von 195-196°C; MS(FAB) = 482.

### 2-(3-Amidinophenyl)-3-(2'-methylsulfonylbiphenyl-4-ylaminocarbonyl)-3-propylcarbazinsäure-tert-butylester 412

0,2 g (0,344 mmol) **411** werden in 5,0 ml Methanol gelöst, mit 0,1 ml Essigsäure und 0,1 g Raney Nickel wasserfeucht versetzt und 18 h unter H₂-Atmosphäre gerührt. Nach üblicher Aufarbeitung erhält man so 0,19 g (100 %) **412** als Kristalle mit einem Schmelzpunkt >300°C; MS(FAB) = 566.

### 1-(3-Amidinophenyl)-4-(2'-methylsulfonylbiphenyl-4-ylamino)-2-propylsemicarbazid 15

0,2 g (0,354 mmol) **412** werden in 10 ml 4N HCl in Dioxan gelöst und 18 h bei RT gerührt. Anch üblicher Aufarbeitung erhält man so 0,13 g (76,9 %) **15** als Kristalle mit einem Schmelzpunkt von >300°C; MS(FAB) = 466.

## Patentansprüche

1. Semicarbazide der allgemeinen Formel I, 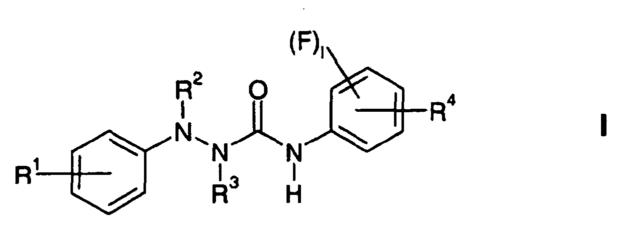 wobei bedeutet:
R¹ -(CH₂)ₙ-NH₂, -CON=C(NH₂)₂, -NHC(=NH)-NH₂ oder -C(=NH)-NH₂, das auch einfach mit -OH, -OCOOA, -OCOO(CH₂)ₙN(A)₂, -OCOO(CH₂)ₘ- Het, -CO-C(A)₂-R⁵, -COOA, -COSA, -COOAr, -COOAr' oder durch 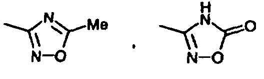 substituiert sein kann,
R² COOA oder H,
R³ unverzweigtes, verzweigtes oder cyclisches Alkyl mit 1-20 C-Atomen, worin eine oder zwei CH₂-Gruppen durch O- oder S-Atome ersetzt sein können, Ar, Ar', X oder Hal,
R⁴ mit S(O)ₖA, S(O)ₖNHA, CF₃, COOA, CH₂NHA, CN oder OA monosubstituiertes Phenyl,
R⁵ -CHal₃, -O(C=O)A oder 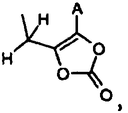
Ar unsubstituiertes oder ein-, zwei- oder dreifach durch A, OH, OA, NH₂, NHA, NA₂, NO₂, CF₃, CN, Hal, NHCOA, COOA, CONH₂, CONHA, CONA₂, S(O)ₙA, S(O)ₙNH₂, S(O)ₙNHA, S(O)ₙNA₂ substituiertes Phenyl oder Naphthyl,
Ar' -(CH₂)ₙ-Ar,
Het einen ein- oder zweikernigen gesättigten, ungesättigten oder aromatischen Heterocyclus mit 1 bis 4 N-, O- und/oder S-Atomen, über N oder C gebunden, der unsubstituiert oder durch A substituiert sein kann,
A H, unverzweigtes, verzweigtes oder cyclisches Alkyl mit 1-20 C-Atomen,
X -(CH₂)ₙ-Y,
Y COOA, 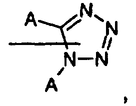
Hal F, Cl, Br oder I,
n 1, 2, 3, 4, 5 oder 6,
m 0 oder 1,
k 0, 1 oder 2,
l 0, 1, 2, 3 oder 4,
sowie ihre pharmazeutisch verträglichen Salze und Solvate.

2. Semicarbazide nach Anspruch 1 der Formel **II** 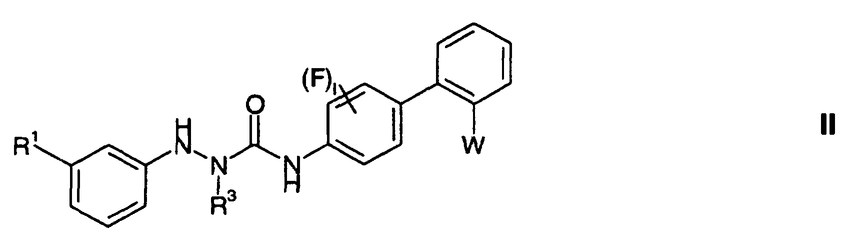 wobei R¹, R³, A und k die in Anspruch 1 angegebene Bedeutung aufweisen, W S(O)ₖA, S(O)ₖNHA, CF₃, COOA, CH₂NHA, CN oder OA und l 0,1 oder 2 ist.

3. Verbindungen nach Anspruch 1 oder 2, nämlich:
4'-[3-(3-Amidinophenyl)-2-propylcarbazoylamino]-biphenyl-2-sulfonamid (1),
4'-[3-(3-(N²-Hydroxyamidino)-phenyl)-2-propylcarbazoylamino]-biphenyl-2-sulfonamid (2),
4'-[3-(3-Amidinophenyl)-2-methylcarbazoylamino]-biphenyl-2-sulfonamid (3),
1-(3-Amidinophenyl)-2-methyl-4-(2'-methylsulfonylbiphenyl-4-yl)-semicarbazid (4),
4'-[3-(3-Amidinophenyl)-2-ethylcarbazoylamino]-biphenyl-2-sulfonamid (5),
1-(3-Amidinophenyl)-2-ethyl-4-(2'-methylsulfonylbiphenyl-4-yl)-semicarbazid (6),
4'-[3-(3-Amidinophenyl)-2-isopropylcarbazoylamino]-biphenyl-2-sulfonamid (7),
1-(3-Amidinophenyl)-2-isopropyl-4-(2'-methylsulfonylbiphenyl-4-yl)-semicarbazid (8),
4'-[3-(3-Amidinophenyl)-2-butylcarbazoylamino]-biphenyl-2-sulfonamid (9),
1-(3-Amidinophenyl)-2-butyl-4-(2'-methylsulfonylbiphenyl-4-yl)-semicarbazid (10),
4'-[3-(3-Amidinophenyl)-2-isobutylcarbazoylamino]-biphenyl-2-sulfonamid (11),
1-(3-Amidinophenyl)-2-isobutyl-4-(2'-methylsulfonylbiphenyl-4-yl)-semicarbazid (12),
4'-[3-(3-Amidinophenyl)-2-pentylcarbazoylamino]-biphenyl-2-sulfonamid (13),
1-(3-Amidinophenyl)-4-(2'-methylsulfonylbiphenyl-4-ylamino)-2-pentylsemicarbazid (14),
1-(3-Amidinophenyl)-4-(2'-methylsulfonylbiphenyl-4-ylamino)-2-propylsemicarbazid (15),
4'-[3-(3-Amidinophenyl)-2-(2-butyl)-carbazoylamino]-biphenyl-2-sulfonamid (16),
1-(3-Amidinophenyl)-2-(2-butyl)-4-(2'-methylsulfonylbiphenyl-4-yl)-semicarbazid(17),
4'-[3-(3-Amidinophenyl)-2-benzylcarbazoylamino]-biphenyl-2-sulfonamid (20),
1-(3-Amidinophenyl)-2-benzyl-4-(2'-methylsulfonylbiphenyl-4-yl)-semicarbazid (21),
1-(3-N²-Hydroxyamidinophenyl)-2-benzyl-4-(2'-methylsulfonylbiphenyl-4-yl)-semicarbazid (22),
4'-[3-(3-Amidinophenyl)-2-phenylcarbazoylamino]-biphenyl-2-sulfonamid (23),
1-(3-Amidinophenyl)-4-(2'-methylsulfonylbiphenyl-4-yl)-2-phenylsemicarbazid (24),
N-[3-[2-Benzyl-4-(2'-methylsulfonylbiphenyl-4-yl)-semicarbazido]-phenyl-(iminomethyl)]-carbaminsäuremethylester (25),
N-[3-[2-Benzyl-4-(2'-methylsulfonylbiphenyl-4-yl)-semicarbazido]-phenyl-(iminomethyl)]-thiocarbaminsäure-S-ethylester (27),
N-[3-[2-Benzyl-4-(2'-methylsulfonylbiphenyl-4-yl)-semicarbazido]-phenyl-(iminomethyl)]-carbaminsäure-(4-methoxybenzylester) (28),
N-[3-[2-Benzyl-4-(2'-methylsulfonylbiphenyl-4-yl)-semicarbazido]-phenyl-(iminomethyl)]-carbaminsäureethylester (29),
N-[3-[2-Benzyl-4-(2'-methylsulfonylbiphenyl-4-yl)-semicarbazido]-phenyl-(iminomethyl)]-carbaminsäurepropylester (30),
N-[3-[2-Benzyl-4-(2'-methylsulfonylbiphenyl-4-yl)-semicarbazido]-phenyl-(iminomethyl)]-carbaminsäurebutylester (31),
N-[3-[2-Benzyl-4-(2'-methylsulfonylbiphenyl-4-yl)-semicarbazido]-phenyl-(iminomethyl)]-carbaminsäureisopropylester (32),
N-[3-[2-Benzyl-4-(2'-methylsulfonylbiphenyl-4-yl)-semicarbazido]-phenyl-(iminomethyl)]-carbaminsäureisobutylester (33),
N-[3-[2-Benzyl-4-(2'-methylsulfonylbiphenyl-4-yl)-semicarbazido]-phenyl-(iminomethyl)]-carbaminsäurephenylester (35),
N-[3-[2-Benzyl-4-(2'-methylsulfonylbiphenyl-4-yl)-semicarbazido]-phenyl-(iminomethyl)]-carbaminsäure-(2-fluorphenylester) (36),
1-(3-Amidinophenyl)-2-(2-fluorbenzyl)-4-(2'-methylsulfonylbiphenyl-4-yl)-semicarbazid (48),
1-(3-Amidinophenyl)-2-(2-methylbenzyl)-4-(2'-methylsulfonylbiphenyl-4-yl)-semicarbazid (49),
1-(3-Amidinophenyl)-2-(2-chlorbenzyl)-4-(2'-methylsulfonylbiphenyl-4-yl)-semicarbazid (50),
1-(3-Amidinophenyl)-2-(3-chlorbenzyl)-4-(2'-methylsulfonylbiphenyl-4-yl)-semicarbazid (51),
4-[1-(3-Amidinophenylamino)-3-(2'-methylsulfonylbiphenyl-4-yl)-ureidomethyl]-benzoesäure (52),
1-(3-Amidinophenyl)-2-(3-methylbenzyl)-4-(2'-methylsulfonylbiphenyl-4-yl)-semicarbazid (53),
1-(3-Amidinophenyl)-4-(2'-methylsulfonylbiphenyl-4-yl)-2-(3-trifluormethylbenzyl)-semicarbazid (54),
1-(3-Amidinophenyl)-4-(2'-methylsulfonylbiphenyl-4-yl)-2-(4-trifluormethylbenzyl)-semicarbazid (55),
1-(3-Amidinophenyl)-4-(2'-methylsulfonylbiphenyl-4-yl)-2-(3-trifluormethoxybenzyl)-semicarbazid (56),
1-(3-Amidinophenyl)-2-benzyl-4-(2'-methylsulfonyl-3-fluor-biphenyl-4-yl)-semicarbazid (57),
1-(3-Amidinophenyl)-2-(3-trifluormethylbenzyl)-4-(2'methylsulfonyl-3-fluor-biphenyl-4-yl)-semicarbazid (58),
1-(3-Amidinophenyl)-2-(4-trifluormethylbenzyl)-4-(2'methylsulfonyl-3-fluor-biphenyl-4-yl)-semicarbazid (59),
1-(3-Amidinophenyl)-2-(2-trifluormethylbenzyl)-4-(2'-methylsulfonylbiphenyl-4-yl)-semicarbazid (60),
1-(3-Amidinophenyl)-2-(2-trifluormethylbenzyl)-4-(2'-methylsulfonyl-3-fluor-biphenyl-4-yl)-semicarbazid (61),
1-(3-Amidinophenyl)-2-benzyl-4-(2'-methylsulfonyl-3,5-difluor-biphenyl-4-yl)-semicarbazid (62),
1-(3-Amidinophenyl)-2-(2-trifluormethylbenzyl)-4-(2'-methylsulfonyl-3,5-difluor-biphenyl-4-yl)-semicarbazid (63),
1-(3-Amidinophenyl)-2-(3-trifluormethylbenzyl)-4-(2'-methylsulfonyl-3,5-difluor-biphenyl-4-yl)-semicarbazid (64),
1-(3-Amidinophenyl)-2-(4-trifluormethylbenzyl)-4-(2'-methylsulfonyl-3,5-difluor-biphenyl-4-yl)-semicarbazid (65),
2-[1-(3-Amidinophenylamino)-3-(2'-sulfamoylbiphenyl-4-yl)-ureido]-essigsäureethylester (66),
3-[1-(3-Amidinophenylamino)-3-(2'-sulfamoylbiphenyl-4-yl)-ureido]-propionsäureethylester (67),
4'-[3-(3-Amidinophenyl)-2-[2-(1-methyltetrazol-5-yl)-ethyl]-carbazoylamino]-biphenyl-2-sulfonamid (68),
4'-[3-(3-Amidinophenyl)-2-(2-methoxyethyl)-carbazoylamino]-biphenyl-2-sulfonamid (69),
4'-[3-(3-Amidinophenyl)-2-(methoxymethyl)-carbazoylamino]-biphenyl-2-sulfonamid (70),
4'-[3-(3-Amidinophenyl)-2-(4-methoxybutyl)-carbazoylamino]-biphenyl-2-sulfonamid (71),
1-(3-Aminomethylphenyl)-2-benzyl-4-(2'-methylsulfonylbiphenyl-4-yl)-semicarbazid (72),
1-(3-Aminomethylphenyl)-4-(2'-methylsulfonylbiphenyl-4-yl)-2-(2-trifluormethylbenzyl)-semicarbazid (73),
1-(3-Aminomethylphenyl)-4-(2'-methylsulfonylbiphenyl-4-yl)-2-(3-trifluormethylbenzyl)-semicarbazid (74),
1-(3-Aminomethylphenyl)-4-(2'-methylsulfonylbiphenyl-4-yl)-2-(4-trifluormethylbenzyl)-semicarbazid (75),
1-(3-Aminomethylphenyl)-2-benzyl-4-(3-fluor-2'-methylsulfonylbiphenyl-4-yl)-semicarbazid (76),
1-(3-Aminomethylphenyl)-4-(3-fluor-2'-methylsulfonylbiphenyl-4-yl)-2-(2-trifluormethylbenzyl)-semicarbazid (77),
1-(3-Aminomethylphenyl)-4-(3-fluor-2'-methylsulfonylbiphenyl-4-yl)-2-(3-trifluormethylbenzyl)-semicarbazid (78),
1-(3-Aminomethylphenyl)-4-(3-fluor-2'-methylsulfonylbiphenyl-4-yl)-2-(4-trifluormethylbenzyl)-semicarbazid (79),
1-(3-Aminomethylphenyl)-2-(3-trifluormethylbenzyl)-4-(2'-methylsulfonylbiphenyl-4-yl)-semicarbazid (80),
1-(3-Aminomethylphenyl)-2-(4-trifluormethylbenzyl)-4-(2'-methylsulfonylbiphenyl-4-yl)-semicarbazid (81),
1-(3-Aminomethylphenyl)-2-benzyl-4-(2'methylsulfonyl-3-fluor-biphenyl-4-yl)-semicarbazid (82),
1-(3-Aminomethylphenyl)-2-(3-trifluormethylbenzyl)-4-(2'-methylsulfonyl-3-fluor-biphenyl-4-yl)-semicarbazid (83),
1-(3-Aminomethylphenyl)-2-benzyl-4-(2'-methylsulfonylbiphenyl-4-yl)-semicarbazid (84),
1-(3-Aminomethylphenyl)-2-(2-trifluormethylbenzyl)-4-(2'-methylsulfonyl-3-fluor-biphenyl-4-yl)-semicarbazid (85),
1-(3-Aminomethylphenyl)-2-benzyl-4-(2'-methylsulfonyl-3,5-difluor-biphenyl-4-yl)-semicarbazid (86),
1-(3-Aminomethylphenyl)-2-(2-trifluormethylbenzyl)-4-(2'-methylsulfonyl-3,5-difluor-biphenyl-4-yl)-semicarbazid (87),
1-(3-Aminomethylphenyl)-2-(4-trifluormethylbenzyl)-4-(2'-methylsulfonyl-3,5-difluor-biphenyl-4-yl)-semicarbazid (88),
1-[3-(N-Hydroxyamidino)-phenyl]-2-(3-trifluormethylbenzyl)-4-(2'-methylsulfonylbiphenyl-4-yl)-semicarbazid (94),
1-[3-(N-Hydroxyamidino)-phenyl]-2-(4-trifluormethylbenzyl)-4-(2'-methylsulfonylbiphenyl-4-yl)-semicarbazid (95),
1-[3-(N-Hydroxyamidino)-phenyl]-2-benzyl-4-(2'-methylsulfonyl-3-fluorbiphenyl-4-yl)-semicarbazid (96),
1-[3-(N-Hydroxyamidino)-phenyl]-2-(3-trifluormethylbenzyl)-4-(2'-methylsulfonyl-3-fluorbiphenyl-4-yl)-semicarbazid (97),
1-[3-(N-Hydroxyamidino)-phenyl]-2-(4-trifluormethylbenzyl)-4-(2'-methylsulfonyl-3-fluorbiphenyl-4-yl)-semicarbazid (98),
1-[3-(N-Hydroxyamidino)-phenyl]-2-(2-trifluormethylbenzyl)-4-(2'-methylsulfonylbiphenyl-4-yl)-semicarbazid (99),
1-[3-(N-Hydroxyamidino)-phenyl]-2-(2-trifluormethylbenzyl)-4-(2'-methylsulfonyl-3-fluorbiphenyl-4-yl)-semicarbazid (100),
1-[3-(N-Hydroxyamidino)-phenyl]-2-benzyl-4-(2'-methylsulfonyl-3,5-difluor-biphenyl-4-yl)-semicarbazid (101),
1-[3-(N-Hydroxyamidino)-phenyl]-2-(2-trifluormethylbenzyl)-4-(2'-methylsulfonyl-3,5-difluor-biphenyl-4-yl)-semicarbazid (102),
1-[3-(N-Hydroxyamidino)-phenyl]-2-(3-trifluormethylbenzyl)-4-(2-methylsulfonyl-3,5-difluor-biphenyl-4-yl)-semicarbazid (103),
1-[3-(N-Hydroxyamidino)-phenyl]-2-(4-trifluormethylbenzyl)-4-(2-methylsulfonyl-3,5-difluor-biphenyl-4-yl)-semicarbazid (104).

4. Verbindungen der Formel **I** gemäß Anspruch 1 und/oder ihre physiologisch unbedenklichen Salze oder Solvate als Arzneimittel.

5. Verwendung von Verbindungen der Formel **I** gemäß Anspruch 1 und/oder ihrer physiologisch unbedenklichen Salze oder Solvate zur Herstellung eines Arzneimittels zur Bekämpfung von Thrombosen, myokardialem Infarkt, Arteriosklerose, Entzündungen, Apoplexie, Angina pectoris, Restenose nach Angioplastie, Claudicatio intermittens, Tumoren, Tumorerkrankungen und/oder Tumormetastasen.

6. Pharmazeutische Zubereitung, umfassend mindestens eine Verbindung der Formel **I** gemäß Anspruch 1 und/oder eines ihrer physiologisch unbedenklichen Salze oder Solvate.

7. Semicarbazidderivate ausgewählt aus der Gruppe
4'-[3-(3-Amidinophenyl)-2-(cyclohexylmethyl)-carbazoylamino]-biphenyl-2-sulfonamid (18),
1-(3-Amidinophenyl)-2-(cyclohexylmethyl)-4-(2'-methylsulfonylbiphenyl-4-yl)-semicarbazid (19),
N-[3-[2-Benzyl-4-(2'-methylsulfonylbiphenyl-4-yl)-semicarbazido]-phenyl-(iminomethyl)]-carbaminsäure-(2,2,2-trichlorethylester) (26),
N-[3-[2-Benzyl-4-(2'-methylsulfonylbiphenyl-4-yl)-semicarbazido]-phenyl-(iminomethyl)]-carbaminsäureallylester (34),
2-Benzyl-1-[3-(N¹-(methylcarboxy)-amidino)-phenyl]-4-(2'-methylsulfonylbiphenyl-4-yl)-semicarbazid (37),
2-Benzyl-4-(2'-methylsulfonylbiphenyl-4-yl)-1-[3-(N¹-(phenylcarboxy)-amidino)-phenyl]-semicarbazid (38),
2-Benzyl-1-[3-(N¹-(isobutylcarboxy)-amidino)-phenyl]-4-(2'-methylsulfonylbiphenyl-4-yl)-semicarbazid (39),
N-[3-[2-Benzyl-4-(2'-methylsulfonylbiphenyl-4-yl)-semicarbazido]-phenyl-(iminomethyl)]-carbaminsäure-(1-methyl-4-piperidinylester) (42),
N-[3-[2-Benzyl-4-(2'-methylsulfonylbiphenyl-4-yl)-semicarbazido]-phenyl-(iminomethyl)]-carbaminsäure-[2-(4-pyridyl)-ethylester] (43),
N-[3-[2-Benzyl-4-(2'-methylsulfonylbiphenyl-4-yl)-semicarbazido]-phenyl-(iminomethyl)]-carbaminsäure-(5-methyl-2-oxo-1,3-dioxol-4-ylmethylester) (44),
N-[3-[2-Benzyl-4-(2'-methylsulfonylbiphenyl-4-yl)-semicarbazido]-phenyl-(iminomethyl)]-carbaminsäure-[2-(3-pyridyl)-ethylester] (45),
N-[3-[2-Benzyl-4-(2'-methylsulfonylbiphenyl-4-yl)-semicarbazido]-phenyl-(iminomethyl)]-carbaminsäure-[2-(N,N-diethylamino)-ethylester] (46),
N-[3-[2-Benzyl-4-(2'-methylsulfonylbiphenyl-4-yl)-semicarbazido]-phenyl-(iminomethyl)]-carbaminsäure-[2-(N-morpholinyl)-ethylester] (47),
2-Benzyl-1-[3-(N¹-ethoxy)-amidino)-phenyl]-4-(2'-methylsulfonylbiphenyl-4-yl)-semicarbazid (90),
2-Benzyl-1-[3-(N¹-vinyloxy)-amidino)-phenyl]-4-(2'-methylsulfonylbiphenyl-4-yl)-semicarbazid (91),
2-Benzyl-1-[3-(N¹-benzyloxy)-amidino)-phenyl]-4-(2'-methylsulfonylbiphenyl-4-yl)-semicarbazid (92),
2-Benzyl-1-[3-(N¹-isopropoxy)-amidino)-phenyl]-4-(2'-methylsulfonylbiphenyl-4-yl)-semicarbazid (93), 2-Benzyl-1-[3-[N¹-(2-methylcarboxy-2-propoxycarbonyl)-amidino]-phenyl]-4-(2'-methylsulfonylbiphenyl-4-yl)-semicarbazid (40),
2-Benzyl-1-[3-[N¹-(1-(methylcarboxy)-ethoxycarbonyl)-amidino]-phenyl]-4-(2'-methylsulfonylbiphenyl-4-yl)-semicarbazid (41),
2-Benzyl-1-[3-(N¹-methoxy)-amidino)-phenyl]-4-(2'-methylsulfonylbiphenyl-4-yl)-semicarbazid (89),
sowie ihre pharmazeutisch verträglichen Salze und Solvate.

## Claims

1. Semicarbazides of the general formula **I**, 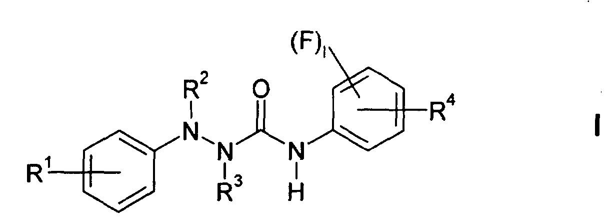 where:
R¹ denotes -(CH₂)ₙ-NH₂, -CON=C(NH₂)₂, -NHC(=NH)-NH₂ or -C(=NH)-NH₂, which may also be monosubstituted by -OH, -OCOOA, -OCOO(CH₂)ₙN(A)₂, -OCOO(CH₂)ₘ-Het, -CO-C(A)₂-R⁵, -COOA, -COSA, -COOAr, -COOAr' or by 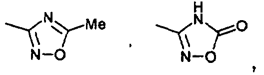
R² denotes COOA or H,
R³ denotes unbranched, branched or cyclic alkyl having 1-20 C atoms, in which one or two CH₂ groups may be replaced by O or S atoms, or denotes Ar, Ar', X or Hal,
R⁴ denotes phenyl which is monosubstituted by S(O)ₖA, S(O)ₖNHA, CF₃, COOA, CH₂NHA, CN or OA,
R⁵ denotes -CHal₃, -O(C=O)A or 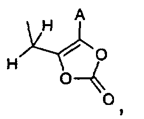
Ar denotes phenyl or naphthyl, each of which is unsubstituted or mono-, di- or trisubstituted by A, OH, OA, NH₂, NHA, NA₂, NO₂, CF₃, CN, Hal, NHCOA, COOA, CONH₂, CONHA, CONA₂, S(O)ₙA, S(O)ₙNH₂, S(O)ₙNHA, S(O)ₙNA₂,
Ar' denotes -(CH₂)ₙ-Ar,
Het denotes a mono- or bicyclic, saturated, unsaturated or aromatic heterocycle having 1 to 4 N, O and/or S atoms, bonded via N or C, which may be unsubstituted or substituted by A,
A denotes H, unbranched, branched or cyclic alkyl having 1-20 C atoms,
X denotes -(CH₂)ₙ-Y,
Y denotes COOA, 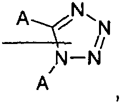
Hal denotes F, Cl, Br or I,
n denotes 1, 2, 3, 4, 5 or 6,
m denotes 0 or 1,
k denotes 0, 1 or 2,
I denotes 0, 1, 2, 3 or 4,
and pharmaceutically tolerated salts and solvates thereof.

2. Semicarbazides according to Claim 1 of the formula II 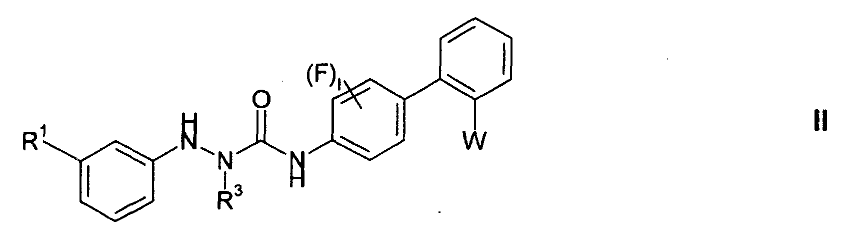 where R¹, R³, A and k have the meaning indicated in Claim 1, W is S(O)ₖA, S(O)ₖNHA, CF₃, COOA, CH₂NHA, CN or OA, and I is 0, 1 or 2.

3. Compounds according to Claim 1 or 2, namely:
4'-[3-(3-amidinophenyl)-2-propylcarbazoylamino]biphenyl-2-sulfonamide (1),
4'-[3-(3-(N²-hydroxyamidino)phenyl)-2-propylcarbazoylamino]biphenyl-2-sulfonamide (2),
4'-[3-(3-amidinophenyl)-2-methylcarbazoylamino]biphenyl-2-sulfonamide (3),
1-(3-amidinophenyl)-2-methyl-4-(2'-methylsulfonylbiphenyl-4-yl)semicarbazide (4),
4'-[3-(3-amidinophenyl)-2-ethylcarbazoylamino]biphenyl-2-sulfonamide (5),
1-(3-amidinophenyl)-2-ethyl-4-(2'-methylsulfonylbiphenyl-4-yl)semicarbazide (6),
4'-[3-(3-amidinophenyl)-2-isopropylcarbazoylamino]biphenyl-2-sulfonamide (7),
1-(3-amidinophenyl)-2-isopropyl-4-(2'-methylsulfonylbiphenyl-4-yl)semicarbazide (8),
4'-[3-(3-amidinophenyl)-2-butylcarbazoylamino]biphenyl-2-sulfonamide (9),
1-(3-amidinophenyl)-2-butyl-4-(2'-methylsulfonylbiphenyl-4-yl)semicarbazide (10),
4'-[3-(3-amidinophenyl)-2-isobutylcarbazoylamino]biphenyl-2-sulfonamide (11),
1-(3-amidinophenyl)-2-isobutyl-4-(2'-methylsulfonylbiphenyl-4-yl)semicarbazide (12),
4'-[3-(3-amidinophenyl)-2-pentylcarbazoylamino]biphenyl-2-sulfonamide (13),
1-(3-amidinophenyl)-4-(2'-methylsulfonylbiphenyl-4-ylamino)-2-pentylsemicarbazide (14),
1-(3-amidinophenyl)-4-(2'-methylsulfonylbiphenyl-4-ylamino)-2-propylsemicarbazide (15),
4'-[3-(3-amidinophenyl)-2-(2-butyl)carbazoylamino]biphenyl-2-sulfonamide (16),
1-(3-amidinophenyl)-2-(2-butyl)-4-(2'-methylsulfonylbiphenyl-4-yl)semicarbazide (17),
4'-[3-(3-amidinophenyl)-2-benzylcarbazoylamino]biphenyl-2-sulfonamide (20),
1-(3-amidinophenyl)-2-benzyl-4-(2'-methylsulfonylbiphenyl-4-yl)semicarbazide (21),
1-(3-N²-hydroxyamidinophenyl)-2-benzyl-4-(2'-methylsulfonylbiphenyl-4-yl)semicarbazide (22),
4'-[3-(3-amidinophenyl)-2-phenylcarbazoylamino]biphenyl-2-sulfonamide (23),
1-(3-amidinophenyl)-4-(2'-methylsulfonylbiphenyl-4-yl)-2-phenylsemicarbazide (24),
methyl N-[3-[2-benzyl-4-(2'-methylsulfonylbiphenyl-4-yl)semicarbazido]-phenyl(iminomethyl)]carbamate (25),
S-ethyl N-[3-[2-benzyl-4-(2'-methylsulfonylbiphenyl-4-yl)semicarbazido]-phenyl(iminomethyl)]thiocarbamate (27),
4-methoxybenzyl N-[3-[2-benzyl-4-(2'-methylsulfonylbiphenyl-4-yl)semicarbazido]phenyl(iminomethyl)]carbamate (28),
ethyl N-[3-[2-benzyl-4-(2'-methylsulfonylbiphenyl-4-yl)semicarbazido]-phenyl(iminomethyl)]carbamate (29),
propyl N-[3-[2-benzyl-4-(2'-methylsulfonylbiphenyl-4-yl)semicarbazido]-phenyl(iminomethyl)]carbamate (30),
butyl N-[3-[2-benzyl-4-(2'-methylsulfonylbiphenyl-4-yl)semicarbazido]-phenyl(iminomethyl)]carbamate (31),
isopropyl N-[3-[2-benzyl-4-(2'-methylsulfonylbiphenyl-4-yl)semicarbazido]phenyl(iminomethyl)]carbamate (32),
isobutyl N-[3-[2-benzyl-4-(2'-methylsulfonylbiphenyl-4-yl)semicarbazido]phenyl(iminomethyl)]carbamate (33),
phenyl N-[3-[2-benzyl-4-(2'-methylsulfonylbiphenyl-4-yl)semicarbazido]-phenyl(iminomethyl)]carbamate (35),
2-fluorophenyl N-[3-[2-benzyl-4-(2'-methylsulfonylbiphenyl-4-yl)semicarbazido]phenyl(iminomethyl)]carbamate (36),
1-(3-amidinophenyl)-2-(2-fluorobenzyl)-4-(2'-methylsulfonylbiphenyl-4-yl)semicarbazide (48),
1-(3-amidinophenyl)-2-(2-methylbenzyl)-4-(2'-methylsulfonylbiphenyl-4-yl)semicarbazide (49),
1-(3-amidinophenyl)-2-(2-chlorobenzyl)-4-(2'-methylsulfonylbiphenyl-4-yl)semicarbazide (50),
1-(3-amidinophenyl)-2-(3-chlorobenzyl)-4-(2'-methylsulfonylbiphenyl-4-yl)semicarbazide (51),
4-[1-(3-amidinophenylamino)-3-(2'-methylsulfonylbiphenyl-4-yl)ureidomethyl]benzoic acid (52),
1-(3-amidinophenyl)-2-(3-methylbenzyl)-4-(2'-methylsulfonylbiphenyl-4-yl)semicarbazide (53),
1-(3-amidinophenyl)-4-(2'-methylsulfonylbiphenyl-4-yl)-2-(3-trifluoromethylbenzyl)semicarbazide (54),
1-(3-amidinophenyl)-4-(2'-methylsulfonylbiphenyl-4-yl)-2-(4-trifluoromethylbenzyl)semicarbazide (55),
1-(3-amidinophenyl)-4-(2'-methylsulfonylbiphenyl-4-yl)-2-(3-trifluoromethoxybenzyl)semicarbazide (56),
1-(3-amidinophenyl)-2-benzyl-4-(2'-methylsulfonyl-3-fluorobiphenyl-4-yl)semicarbazide (57),
1-(3-amidinophenyl)-2-(3-trifluoromethylbenzyl)-4-(2'-methylsulfonyl-3-fluorobiphenyl-4-yl)semicarbazide (58),
1-(3-amidinophenyl)-2-(4-trifluoromethylbenzyl)-4--(2'-methylsulfonyl-3-fluor-biphenyl-4-yl)semicarbazide (59),
1-(3-amidinophenyl)-2-(2-trifluoromethylbenzyl)-4-(2'-methylsulfonylbiphenyl-4-yl)semicarbazide (60),
1-(3-amidinophenyl)-2-(2-trifluoromethylbenzyl)-4-(2'-methylsulfonyl-3-fluor-biphenyl-4-yl)semicarbazide (61),
1-(3-amidinophenyl)-2-benzyl-4-(2'-methylsulfonyl-3,5-difluor-biphenyl-4-yl)semicarbazide (62),
1-(3-amidinophenyl)-2-(2-trifluoromethylbenzyl)-4-(2'-methylsulfonyl-3,5-difluor-biphenyl-4-yl)semicarbazide (63),
1-(3-amidinophenyl)-2-(3-trifluoromethylbenzyl)-4-(2'-methylsulfonyl-3,5-difluor-biphenyl-4-yl)semicarbazide (64),
1-(3-amidinophenyl)-2-(4-trifluoromethylbenzyl)-4-(2'-methylsulfonyl-3,5-difluor-biphenyl-4-yl)semicarbazide (65),
ethyl 2-[1-(3-amidinophenylamino)-3-(2'-sulfamoylbiphenyl-4-yl)ureido]-acetate (66),
ethyl 3-[1-(3-amidinophenylamino)-3-(2'-sulfamoylbiphenyl-4-yl)ureido]-propionate (67),
4'-[3-(3-amidinophenyl)-2-[2-(1-methyltetrazol-5-yl)ethyl]carbazoylamino]biphenyl-2-sulfonamide (68),
4'-[3-(3-amidinophenyl)-2-(2-methoxyethyl)carbazoylamino]biphenyl-2-sulfonamide (69),
4'-[3-(3-amidinophenyl)-2-(methoxymethyl)carbazoylamino]biphenyl-2-sulfonamide (70),
4'-[3-(3-amidinophenyl)-2-(4-methoxybutyl)carbazoylamino]biphenyl-2-sulfonamide (71),
1-(3-aminomethylphenyl)-2-benzyl-4-(2'-methylsulfonylbiphenyl-4-yl)semicarbazide (72),
1-(3-aminomethylphenyl)-4-(2'-methylsulfonylbiphenyl-4-yl)-2-(2-trifluoromethylbenzyl)semicarbazide (73),
1-(3-aminomethylphenyl)-4-(2'-methylsulfonylbiphenyl-4-yl)-2-(3-trifluoromethylbenzyl)semicarbazide (74),
1-(3-aminomethylphenyl)-4-(2'-methylsulfonylbiphenyl-4-yl)-2-(4-trifluoromethylbenzyl)semicarbazide (75),
1-(3-aminomethylphenyl)-2-benzyl-4-(3-fluoro-2'-methylsulfonylbiphenyl-4-yl)semicarbazide (76),
1-(3-aminomethylphenyl)-4-(3-fluoro-2'-methylsulfonylbiphenyl-4-yl)-2-(2-trifluoromethylbenzyl)semicarbazide (77),
1-(3-aminomethylphenyl)-4-(3-fluoro-2'-methylsulfonylbiphenyl-4-yl)-2-(3-trifluoromethylbenzyl)semicarbazide (78),
1-(3-aminomethylphenyl)-4-(3-fluoro-2'-methylsulfonylbiphenyl-4-yl)-2-(4-trifluoromethylbenzyl)semicarbazide (79),
1-(3-aminomethylphenyl)-2-(3-trifluoromethylbenzyl)-4-(2'-methylsulfonylbiphenyl-4-yl)semicarbazide (80),
1-(3-aminomethylphenyl)-2-(4-trifluoromethylbenzyl)-4-(2'-methylsulfonylbiphenyl-4-yl)semicarbazide (81),
1-(3-aminomethylphenyl)-2-benzyl-4-(2'-methylsulfonyl-3-fluorobiphenyl-4-yl)semicarbazide (82),
1-(3-aminomethylphenyl)-2-(3-trifluoromethylbenzyl)-4-(2'-methylsulfonyl-3-fluorobiphenyl-4-yl)semicarbazide (83),
1-(3-aminomethylphenyl)-2-benzyl-4-(2'-methylsulfonylbiphenyl-4-yl)semicarbazide (84),
1-(3-aminomethylphenyl)-2-(2-trifluoromethylbenzyl)-4-(2'-methylsulfonyl-3-fluorobiphenyl-4-yl)semicarbazide (85),
1-(3-aminomethylphenyl)-2-benzyl-4-(2'-methylsulfonyl-3,5-difluorobiphenyl-4-yl)semicarbazide (86),
1-(3-aminomethylphenyl)-2-(2-trifluoromethylbenzyl)-4-(2'-methylsulfonyl-3,5-difluorobiphenyl-4-yl)semicarbazide (87),
1-(3-aminomethylphenyl)-2-(4-trifluoromethylbenzyl)-4-(2'-methylsulfonyl-3,5-difluorobiphenyl-4-yl)semicarbazide (88),
1-[3-(N-hydroxyamidino)phenyl]-2-(3-trifluoromethylbenzyl)-4-(2'-methylsulfonylbiphenyl-4-yl)semicarbazide (94),
1-[3-(N-hydroxyamidino)phenyl]-2-(4-trifluoromethylbenzyl)-4-(2'-methylsulfonylbiphenyl-4-yl)semicarbazide (95),
1-[3-(N-hydroxyamidino)phenyl]-2-benzyl-4-(2'-methylsuffonyl-3-fluorobiphenyl-4-yl)semicarbazide (96),
1-[3-(N-hydroxyamidino)phenyl]-2-(3-trifluoromethylbenzyl)-4-(2'-methylsulfonyl-3-fluorobiphenyl-4-yl)semicarbazide (97),
1-[3-(N-hydroxyamidino)phenyl]-2-(4-trifluoromethylbenzyl)-4-(2'-methylsulfonyl-3-fluorobiphenyl-4-yl)semicarbazide (98),
1-[3-(N-hydroxyamidino)phenyl]-2-(2-trifluoromethylbenzyl)-4-(2'-methylsulfonylbiphenyl-4-yl)semicarbazide (99),
1-[3-(N-hydroxyamidino)phenyl]-2-(2-trifluoromethylbenzyl)-4-(2'-methylsulfonyl-3-fluorobiphenyl-4-yl)semicarbazide (100),
1-[3-(N-hydroxyamidino)phenyl]-2-benzyl-4-(2'-methylsulfonyl-3, 5-difluoro-biphenyl-4-yl)semicarbazide (101),
1-[3-(N-hydroxyamidino)phenyl]-2-(2-trifluoromethylbenzyl)-4-(2'-methylsulfonyl-3,5-difluorobiphenyl-4-yl)semicarbazide (102),
1-[3-(N-hydroxyamidino)phenyl]-2-(3-trifluoromethylbenzyl)-4-(2-methylsulfonyl-3,5-difluorobiphenyl-4-yl)semicarbazide (103),
1-[3-(N-hydroxyamidino)phenyl]-2-(4-trifluoromethylbenzyl)-4-(2-methylsulfonyl-3,5-difluorobiphenyl-4-yl)semicarbazide (104).

4. Compounds of the formula **I** according to Claim 1 and/or physiologically acceptable salts or solvates thereof as medicaments.

5. Use of compounds of the formula **I** according to Claim 1 and/or physiologically acceptable salts or solvates thereof for the preparation of a medicament for combating thromboses, myocardial infarction, arteriosclerosis, inflammation, apoplexy, angina pectoris, restenosis after angioplasty, claudicatio intermittens, tumours, tumour diseases and/or tumour metastases.

6. Pharmaceutical composition comprising at least one compound of the formula **I** according Claim 1 and/or one of its physiologically acceptable salts or solvates.

7. Semicarbazide derivatives selected from the group
4'-[3-(3-amidinophenyl)-2-(cyclohexylmethyl)carbazoylamino]biphenyl-2-sulfonamide (18),
1-(3-amidinophenyl)-2-(cyclohexylmethyl)-4-(2'-methylsulfonylbiphenyl-4-yl)semicarbazide (19),
2,2,2-trichloroethyl N-[3-[2-benzyl-4-(2'-methylsulfonylbiphenyl-4-yl)semicarbazido]phenyl(iminomethyl)]carbamate (26),
allyl N-[3-[2-benzyl-4-(2'-methylsulfonylbiphenyl-4-yl)semicarbazido]phenyl(iminomethyl)]carbamate (34),
2-benzyl-1-[3-(N¹-(methylcarboxy)amidino)phenyl]-4-(2'-methylsulfonylbiphenyl-4-yl)semicarbazide (37),
2-benzyl-4-(2'-methylsulfonylbiphenyl-4-yl)-1-[3-(N¹-(phenylcarboxy)amidino)phenyl]semicarbazide (38),
2-benzyl-1-[3-(N¹-(isobutylcarboxy)amidino)phenyl]-4-(2'-methylsulfonylbiphenyl-4-yl)semicarbazide (39),
1-methyl-4-piperidinyl N-[3-[2-benzyl-4-(2'-methylsulfonylbiphenyl-4-yl)semicarbazido]phenyl(iminomethyl)]carbamate (42),
2-(4-pyridyl)ethyl N-[3-[2-benzyl-4-(2'-methylsulfonylbiphenyl-4-yl)semicarbazido]phenyl(iminomethyl)]carbamate (43),
5-methyl-2-oxo-1,3-dioxol-4-ylmethyl N-[3-[2-benzyl-4-(2'-methylsulfonylbiphenyl-4-yl)semicarbazido]phenyl(iminomethyl)]carbamate (44),
2-(3-pyridyl)ethyl N-[3-[2-benzyl-4-(2'-methylsulfonylbiphenyl-4-yl)semicarbazido]phenyl(iminomethyl)]carbamate (45),
2-(N,N-diethylamino)ethyl N-[3-[2-benzyl-4-(2'-methylsulfonylbiphenyl-4-yl)semicarbazido]phenyl(iminomethyl)]carbamate (46),
2-(N-morpholinyl)ethyl N-[3-[2-benzyl-4-(2'-methylsulfonylbiphenyl-4-yl)semicarbazido]phenyl(iminomethyl)]carbamate (47),
2-benzyl-1-[3-(N¹-ethoxy)amidino)-phenyl]-4-(2'-methylsulfonylbiphenyl-4-yl)semicarbazide (90),
2-benzyl-1-[3-(N¹-vinyloxy)amidino)-phenyl]-4-(2'-methylsulfonylbiphenyl-4-yl)semicarbazide (91),
2-benzyl-1-[3-(N¹-benzyloxy)amidino)-phenyl]-4-(2'-methylsulfonylbiphenyl-4-yl)semicarbazide (92),
2-benzyl-1-[3-(N¹-isopropoxy)amidino)-phenyl]-4-(2'-methylsulfonylbiphenyl-4-yl)semicarbazide (93),
2-benzyl-1-[3-[N¹-(2-methylcarboxy-2-propoxycarbonyl)amidino]phenyl]-4-(2'-methylsulfonylbiphenyl-4-yl)semicarbazide (40),
2-benzyl-1-[3-[N¹-(1-(methylcarboxy)ethoxycarbonyl)amidino]phenyl]-4-(2'-methylsulfonylbiphenyl-4-yl)semicarbazide (41),
2-benzyl-1-[3-(N¹-methoxy)-amidino)phenyl]-4-(2'-methylsulfonylbiphenyl-4-yl)semicarbazide (89),
and pharmaceutically tolerated salts and solvates thereof.

## Revendications

1. Semicarbazides de la formule générale **I**, 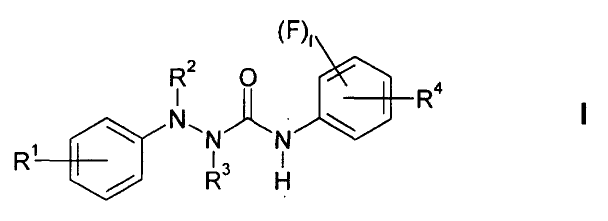 dans laquelle:
R¹ représente -(CH₂)ₙ-NH₂, -CON=C(NH₂)₂, -NHC(=NH)-NH₂ ou -C(=NH)-NH₂, lequel peut également être monosubstitué par -OH, -OCOOA, -OCOO(CH₂)ₙN(A)₂, -OCOO(CH₂)ₘ-Het, -CO-C(A)₂-R⁵, -COOA, -COSA, -COOAr, -COOAr' ou par 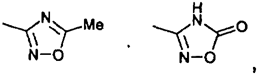
R² représente COOA ou H,
R³ représente un alkyle non ramifié, ramifié ou cyclique comportant de 1 à 20 atomes de C, où un ou deux groupes CH₂ peuvent être remplacés par des atomes de O ou de S, ou représente Ar, Ar', X ou Hal,
R⁴ représente phényle, lequel est monosubstitué par S(O)ₖA, S(O)ₖNHA, CF₃, COOA, CH₂NHA, CN ou OA,
R⁵ représente -CHal₃, -O(C=O)A ou 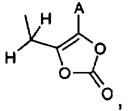
Ar représente phényle ou naphthyle, dont chacun est non substitué ou mono-, di- ou trisubstitué par A, OH, OA, NH₂, NHA, NA₂, NO₂, CF₃, CN, Hal, NHCOA, COOA, CONH₂, CONHA, CONA₂, S(O)ₙA, S(O)ₙNH₂, S(O)ₙNHA, S(O)ₙNA₂,
Ar' représente -(CH₂)ₙ-Ar,
Het représente un hétérocycle mono- ou bicyclique, saturé, insaturé ou aromatique comportant de 1 à 4 atomes de N, de O et/ou de S, lié via N ou C, lequel peut être non substitué ou substitué par A,
A représente H, un alkyle non ramifié, ramifié ou cyclique comportant de 1 à 20 atomes de C,
X représente -(CH₂)ₙ-Y,
Y représente COOA, 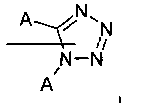
Hal représente F, Cl, Br ou I,
n représente 1, 2, 3, 4, 5 ou 6,
m représente 0 ou 1,
k représente 0, 1 ou 2,
l représente 0, 1, 2, 3 ou 4,
et leurs sels et solvates pharmaceutiquement tolérés.

2. Semicarbazides selon la revendication 1 de la formule **II** 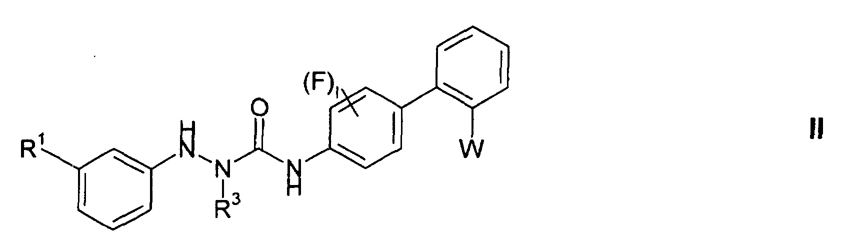 dans laquelle R¹, R³, A et k présentent la signification indiquée dans la revendication 1, W est S(O)ₖA, S(O)ₖNHA, CF₃, COOA, CH₂NHA, CN ou OA, et I est 0, 1 ou 2.

3. Composés selon la revendication 1 ou 2, à savoir:
4'-[3-(3-amidinophényl)-2-propylcarbazoylamino]biphényl-2-sulfonamide (1),
4'-[3-(3-(N²-hydroxyamidino)phényl)-2-propylcarbazoylamino]biphényl-2-sulfonamide (2),
4'-[3-(3-amidinophényl)-2-méthylcarbazoylamino]biphényl-2-sulfonamide (3),
1-(3-amidinophényl)-2-méthyl-4-(2'-méthylsulfonylbiphényl-4-yl)semicarbazide (4),
4'-[3-(3-amidinophényl)-2-éthylcarbazoylamino]biphényl-2-sulfonamide (5),
1-(3-amidinophényl)-2-éthyl-4-(2'-méthylsulfonylbiphényl-4-yl)semicarbazide (6),
4'-[3-(3-amidinophényl)-2-isopropylcarbazoylamino]biphényl-2-sulfonamide (7),
1-(3-amidinophényl)-2-isopropyl-4-(2'-méthylsulfonylbiphényl-4-yl)semicarbazide (8),
4'-[3-(3-amidinophényl)-2-butylcarbazoylamino]biphényl-2-sulfonamide (9),
1-(3-amidinophényl)-2-butyl-4-(2'-méthylsulfonylbiphényl-4-yl)semicarbazide (10),
4'-[3-(3-amidinophényl)-2-isobutylcarbazoylamino]biphényl-2-sulfonamide (11),
1-(3-amidinophényl)-2-isobutyl-4-(2'-méthylsulfonylbiphényl-4-yl)semicarbazide (12),
4'-[3-(3-amidinophényl)-2-pentylcarbazoylamino]biphényl-2-sulfonamide (13),
1-(3-amidinophényl)-4-(2'-méthylsulfonylbiphényl-4-ylamino)-2-pentylsemicarbazide (14),
1-(3-amidinophényl)-4-(2'-méthylsulfonylbiphényl-4-ylamino)-2-propylsemicarbazide (15),
4'-[3-(3-amidinophényl)-2-(2-butyl)carbazoylamino]biphényl-2-sulfonamide (16),
1-(3-amidinophényl)-2-(2-butyl)-4-(2'-méthylsulfonylbiphényl-4-yl)semicarbazide (17),
4'-[3-(3-amidinophényl)-2-benzylcarbazoylamino]biphényl-2-sulfonamide (20),
1-(3-amidinophényl)-2-benzyl-4-(2'-méthylsulfonylbiphényl-4-yl)semicarbazide (21),
1-(3-N²-hydroxyamidinophényl)-2-benzyl-4-(2'-méthylsulfonylbiphényl-4-yl)semicarbazide (22),
4'-[3-(3-amidinophényl)-2-phénylcarbazoylamino]biphényl-2-sulfonamide (23),
1-(3-amidinophényl)-4-(2'-méthylsulfonylbiphényl-4-yl)-2-phénylsemicarbazide (24),
N-[3-[2-benzyl-4-(2'-méthylsulfonylbiphényl-4-yl)semicarbazido]phényl(iminométhyl)]carbamate de méthyle (25),
N-[3-[2-benzyl-4-(2'-méthylsulfonylbiphényl-4-yl)semicarbazido]phényl(iminométhyl)]thiocarbamate de S-éthyle (27),
N-[3-[2-benzyl-4-(2'-méthylsulfonylbiphényl-4-yl)semicarbazido]phényl(iminométhyl)]carbamate de 4-méthoxybenzyle (28),
N-[3-[2-benzyl-4-(2'-méthylsulfonylbiphényl-4-yl)semicarbazido]phényl(iminométhyl)]carbamate d'éthyle (29),
N-[3-[2-benzyl-4-(2'-méthylsulfonylbiphényl-4-yl)semicarbazido]phényl(iminométhyl)]carbamate de propyle (30),
N-[3-[2-benzyl-4-(2'-méthylsulfonylbiphényl-4-yl)semicarbazido]phényl(iminométhyl)]carbamate de butyle (31),
N-[3-[2-benzyl-4-(2'-méthylsulfonylbiphényl-4-yl)semicarbazido]phényl(iminométhyl)]carbamate d'isopropyle (32),
N-[3-[2-benzyl-4-(2'-méthylsulfonylbiphényl-4-yl)semicarbazido]phényl(iminométhyl)]carbamate d'isobutyle (33),
N-[3-[2-benzyl-4-(2'-méthylsulfonylbiphényl-4-yl)semicarbazido]phényl(iminométhyl)]carbamate de phényle (35),
N-[3-[2-benzyl-4-(2'-méthylsulfonylbiphényl-4-yl)semicarbazido]phényl(iminométhyl)]carbamate de 2-fluorophényle (36),
1-(3-amidinophényl)-2-(2-fluorobenzyl)-4-(2'-méthylsulfonylbiphényl-4-yl)semicarbazide (48),
1-(3-amidinophényl)-2-(2-méthylbenzyl)-4-(2'-méthylsulfonylbiphényl-4-yl)semicarbazide (49),
1-(3-amidinophényl)-2-(2-chlorobenzyl)-4-(2'-méthylsulfonylbiphényl-4-yl)semicarbazide (50),
1-(3-amidinophényl)-2-(3-chlorobenzyl)-4-(2'-méthylsulfonylbiphényl-4-yl)semicarbazide (51),
acide 4-[1-(3-amidinophénylamino)-3-(2'-méthylsulfonylbiphényl-4-yl)-uréidométhyl]benzoïque (52),
1-(3-amidinophényl)-2-(3-méthylbenzyl)-4-(2'-méthylsulfonylbiphényl-4-yl)semicarbazide (53),
1-(3-amidinophényl)-4-(2'-méthylsulfonylbiphényl-4-yl)-2-(3-trifluorométhylbenzyl)semicarbazide (54),
1-(3-amidinophényl)-4-(2'-méthylsulfonylbiphényl-4-yl)-2-(4-trifluorométhylbenzyl)semicarbazide (55),
1-(3-amidinophényl)-4-(2'-méthylsulfonylbiphényl-4-yl)-2-(3-trifluorométhoxybenzyl)semicarbazide (56),
1-(3-amidinophényl)-2-benzyl-4-(2'-méthylsulfonyl-3-fluorobiphényl-4-yl)semicarbazide (57),
1-(3-amidinophényl)-2-(3-trifluorométhylbenzyl)-4-(2'-méthylsulfonyl-3-fluorobiphényl-4-yl)semicarbazide (58),
1-(3-amidinophényl)-2-(4-trifluorométhylbenzyl)-4--(2'-méthylsulfonyl-3-fluor-biphényl-4-yl)semicarbazide (59),
1-(3-amidinophényl)-2-(2-trifluorométhylbenzyl)-4-(2'-méthylsulfonylbiphényl-4-yl)semicarbazide (60),
1-(3-amidinophényl)-2-(2-trifluorométhylbenzyl)-4-(2'-méthylsulfonyl-3-fluor-biphényl-4-yl)semicarbazide (61),
1-(3-amidinophényl)-2-benzyl-4-(2'-méthylsulfonyl-3,5-difluor-biphényl-4-yl)semicarbazide (62),
1-(3-amidinophényl)-2-(2-trifluorométhylbenzyl)-4-(2'-méthylsulfonyl-3,5-difluor-biphényl-4-yl)semicarbazide (63),
1-(3-amidinophényl)-2-(3-trifluorométhylbenzyl)-4-(2'-méthylsulfonyl-3,5-difluor-biphényl-4-yl)semicarbazide (64),
1-(3-amidinophényl)-2-(4-trifluorométhylbenzyl)-4-(2'-méthylsulfonyl-3,5-difluor-biphényl-4-yl)semicarbazide (65),
2-[1-(3-amidinophénylamino)-3-(2'-sulfamoylbiphényl-4-yl)uréido]-acétate d'éthyle (66),
3-[1-(3-amidinophénylamino)-3-(2'-sulfamoylbiphényl-4-yl)uréido]-propionate d'éthyle (67),
4'-[3-(3-amidinophényl)-2-[2-(1-méthyltétrazol-5-yl)éthyl]carbazoylamino]biphényl-2-sulfonamide (68),
4'-[3-(3-amidinophényl)-2-(2-méthoxyéthyl)carbazoylamino]biphényl-2-sulfonamide (69),
4'-[3-(3-amidinophényl)-2-(méthoxyméthyl)carbazoylamino]biphényl-2-sulfonamide (70),
4'-[3-(3-amidinophényl)-2-(4-méthoxybutyl)carbazoylamino]biphényl-2-sulfonamide (71),
1-(3-aminométhylphényl)-2-benzyl-4-(2'-méthylsulfonylbiphényl-4-yl)-semicarbazide (72),
1-(3-aminométhylphényl)-4-(2'-méthylsulfonylbiphényl-4-yl)-2-(2-trifluorométhylbenzyl)semicarbazide (73),
1-(3-aminométhylphényl)-4-(2'-méthylsulfonylbiphényl-4-yl)-2-(3-trifluorométhylbenzyl)semicarbazide (74),
1-(3-aminométhylphényl)-4-(2'-méthylsulfonylbiphényl-4-yl)-2-(4-trifluorométhylbenzyl)semicarbazide (75),
1-(3-aminométhylphényl)-2-benzyl-4-(3-fluoro-2'-méthylsulfonylbiphényl-4-yl)semicarbazide (76),
1-(3-aminométhylphényl)-4-(3-fluoro-2'-méthylsulfonylbiphényl-4-yl)-2-(2-trifluorométhylbenzyl)semicarbazide (77),
1-(3-aminométhylphényl)-4-(3-fluoro-2'-méthylsulfonylbiphényl-4-yl)-2-(3-trifluorométhylbenzyl)semicarbazide (78),
1-(3-aminométhylphényl)-4-(3-fluoro-2'-méthylsulfonylbiphényl-4-yl)-2-(4-trifluorométhylbenzyl)semicarbazide (79),
1-(3-aminométhylphényl)-2-(3-trifluorométhylbenzyl)-4-(2'-méthylsulfonylbiphényl-4-yl)semicarbazide (80),
1-(3-aminométhylphényl)-2-(4-trifluorométhylbenzyl)-4-(2'-méthylsulfonylbiphényl-4-yl)semicarbazide (81),
1-(3-aminométhylphényl)-2-benzyl-4-(2'-méthylsulfonyl-3-fluorobiphényl-4-yl)semicarbazide (82),
1-(3-aminométhylphényl)-2-(3-trifluorométhylbenzyl)-4-(2'-méthylsulfonyl-3-fluorobiphényl-4-yl)semicarbazide (83),
1-(3-aminométhylphényl)-2-benzyl-4-(2'-méthylsulfonylbiphényl-4-yl)semicarbazide (84),
1-(3-aminométhylphényl)-2-(2-trifluorométhylbenzyl)-4-(2'-méthylsulfonyl-3-fluorobiphényl-4-yl)semicarbazide (85),
1-(3-aminométhylphényl)-2-benzyl-4-(2'-méthylsulfonyl-3,5-difluorobiphényl-4-yl)semicarbazide (86),
1-(3-aminométhylphényl)-2-(2-trifluorométhylbenzyl)-4-(2'-méthylsulfonyl-3,5-difluorobiphényl-4-yl)semicarbazide (87),
1-(3-aminométhylphényl)-2-(4-trifluorométhylbenzyl)-4-(2'-méthylsulfonyl-3,5-difluorobiphényl-4-yl)semicarbazide (88),
1-[3-(N-hydroxyamidino)phényl]-2-(3-trifluorométhylbenzyl)-4-(2'-méthylsulfonylbiphényl-4-yl)semicarbazide (94),
1-[3-(N-hydroxyamidino)phényl]-2-(4-trifluorométhylbenzyl)-4-(2'-méthylsulfonylbiphényl-4-yl)semicarbazide (95),
1-[3-(N-hydroxyamidino)phényl]-2-benzyl-4-(2'-méthylsulfonyl-3-fluorobiphényl-4-yl)semicarbazide (96),
1-[3-(N-hydroxyamidino)phényl]-2-(3-trifluorométhylbenzyl)-4-(2'-méthylsulfonyl-3-fluorobiphényl-4-yl)semicarbazide (97),
1-[3-(N-hydroxyamidino)phényl]-2-(4-trifluorométhylbenzyl)-4-(2'-méthylsulfonyl-3-fluorobiphényl-4-yl)semicarbazide (98),
1-[3-(N-hydroxyamidino)phényl]-2-(2-trifluorométhylbenzyl)-4-(2'-méthylsulfonylbiphényl-4-yl)semicarbazide (99),
1-[3-(N-hydroxyamidino)phényl]-2-(2-trifluorométhylbenzyl)-4-(2'-méthylsulfonyl-3-fluorobiphényl-4-yl)semicarbazide (100),
1-[3-(N-hydroxyamidino)phényl]-2-benzyl-4-(2'-méthylsulfonyl-3,5-difluoro-biphényl-4-yl)semicarbazide (101),
1-[3-(N-hydroxyamidino)phényl]-2-(2-trifluorométhylbenzyl)-4-(2'-méthylsulfonyl-3,5-difluorobiphényl-4-yl)semicarbazide (102),
1-[3-(N-hydroxyamidino)phényl]-2-(3-trifluorométhylbenzyl)-4-(2-méthylsulfonyl-3,5-difluorobiphényl-4-yl)semicarbazide (103),
1-[3-(N-hydroxyamidino)phényl]-2-(4-trifluorométhylbenzyl)-4-(2-méthylsulfonyl-3,5-difluorobiphényl-4-yl)semicarbazide (104).

4. Composés de la formule **I** selon la revendication 1 et/ou leurs sels ou solvates physiologiquement acceptables en tant que médicaments.

5. Utilisation de composés de la formule **I** selon la revendication 1 et/ou de leurs sels ou solvates physiologiquement acceptables pour la préparation d'un médicament pour combattre les thromboses, l'infarctus du myocarde, l'artériosclérose, les inflammations, l'apoplexie, l'angine de poitrine, la resténose après angioplastie, la claudication intermittente, les tumeurs, les maladies tumorales et/ou les métastases tumorales.

6. Composition pharmaceutique comprenant au moins un composé de la formule **I** selon la revendication 1 et/ou l'un de ses sels ou solvates physiologiquement acceptables.

7. Dérivés de semicarbazides choisis parmi le groupe
4'-[3-(3-amidinophényl)-2-(cyclohexylméthyl)carbazoylamino]biphényl-2-sulfonamide (18),
1-(3-amidinophényl)-2-(cyclohexylméthyl)-4-(2'-méthylsulfonylbiphényl-4-yl)semicarbazide (19),
N-[3-[2-benzyl-4-(2'-méthylsulfonylbiphényl-4-yl)semicarbazido]phényl(iminométhyl)]carbamate de 2,2,2-trichloroéthyle (26),
N-[3-[2-benzyl-4-(2'-méthylsulfonylbiphényl-4-yl)semicarbazido]phényl(iminométhyl)]carbamate d'allyle (34),
2-benzyl-1-[3-(N¹-(méthylcarboxy)amidino)phényl]-4-(2'-méthylsulfonylbiphényl-4-yl)semicarbazide (37),
2-benzyl-4-(2'-méthylsulfonylbiphényl-4-yl)-1-[3-(N¹-(phénylcarboxy)-amidino)phényl]semicarbazide (38),
2-benzyl-1-[3-(N¹-(isobutylcarboxy)amidino)phényl]-4-(2'-méthylsulfonylbiphényl-4-yl)semicarbazide (39),
N-[3-[2-benzyl-4-(2'-méthylsulfonylbiphényl-4-yl)semicarbazido]phényl(iminométhyl)]carbamate de 1-méthyl-4-pipéridinyle (42),
N-[3-[2-benzyl-4-(2'-méthylsulfonylbiphényl-4-yl)semicarbazido]phényl(iminométhyl)]carbamate de 2-(4-pyridyl)éthyle (43),
N-[3-[2-benzyl-4-(2'-méthylsulfonylbiphényl-4-yl)semicarbazido]phényl(iminométhyl)]carbamate de 5-méthyl-2-oxo-1,3-dioxol-4-ylméthyle (44),
N-[3-[2-benzyl-4-(2'-méthylsulfonylbiphényl-4-yl)semicarbazido]phényl(iminométhyl)]carbamate de 2-(3-pyridyl)éthyle (45),
N-[3-[2-benzyl-4-(2'-méthylsulfonylbiphényl-4-yl)semicarbazido]phényl(iminométhyl)]carbamate de 2-(N,N-diéthylamino)éthyle (46),
N-[3-[2-benzyl-4-(2'-méthylsulfonylbiphényl-4-yl)semicarbazido]phényl(iminométhyl)]carbamate de 2-(N-morpholinyl)éthyle (47),
2-benzyl-1-[3-(N¹-éthoxy)amidino)-phényl]-4-(2'-méthylsulfonylbiphényl-4-yl)semicarbazide (90),
2-benzyl-1-[3-(N¹-vinyloxy)amidino)-phényl]-4-(2'-méthylsulfonylbiphényl-4-yl)semicarbazide (91),
2-benzyl-1-[3-(N¹-benzyloxy)amidino)-phényl]-4-(2'-méthylsulfonylbiphényl-4-yl)semicarbazide (92),
2-benzyl-1-[3-(N¹-isopropoxy)amidino)-phényl]-4-(2'-méthylsulfonylbiphényl-4-yl)semicarbazide (93),
2-benzyl-1-[3-[N¹-(2-méthylcarboxy-2-propoxycarbonyl)amidino]phényl)-4-(2'-méthylsulfonylbiphényl-4-yl)semicarbazide (40),
2-benzyl-1-[3-[N¹-(1-(méthylcarboxy)éthoxycarbonyl)amidino]phényl]-4-(2'-méthylsulfonylbiphényl-4-yl)semicarbazide (41 ),
2-benzyl-1-[3-(N¹-méthoxy)-amidino)phényl]-4-(2'-méthylsulfonylbiphényl-4-yl)semicarbazide (89),
et leurs sels et solvates pharmaceutiquement tolérés.
